# EUROPÄISCHE PATENTSCHRIFT

(11) **EP 3 288 439 B1**
(45) Veröffentlichungstag und Bekanntmachung des Hinweises auf die Patenterteilung: **23.06.2021**
(21) Anmeldenummer: 16717645.2
(22) Anmeldetag: 21.04.2016
(51) Int. Cl.: A61B 3/032, A61B 3/00

(54) **VERFAHREN UND DATENVERARBEITUNGSPROGRAMM ZUR BESTIMMUNG DES SEHVERMÖGENS EINES BENUTZERS**
METHOD AND DATA PROCESSING PROGRAM FOR DETERMINING THE VISUAL ABILITY OF A USER
PROCÉDÉ ET PROGRAMME DE TRAITEMENT DES DONNÉES DESTINÉS À LA DÉTERMINATION DE L'APTITUDE VISUELLE D'UN UTILISATEUR

(30) Priorität: 30.04.2015 EP 15166029
(43) Veröffentlichungstag der Anmeldung: 07.03.2018
(73) Patentinhaber: Oculocare Medical AG, 8004 Zürich (CH)
(72) Erfinder: SCHMID, Martin K., 6006 Luzern (CH); THIEL, Michael A., 8309 Nürensdorf (CH); BACHMANN, Lucas M., 8002 Zürich (CH); LIENHARD, Kenny R., 8004 Zürich (CH)
(74) Vertreter: Bremi, Tobias Hans
(86) Internationale Anmeldenummer: PCT/EP2016/058911
(87) Internationale Veröffentlichungsnummer: WO 2016/173924

(56) Entgegenhaltungen:
- US-A- 4 798 456
- US-A1- 2005 122 477
- US-A1- 2012 050 685

## Beschreibung

### TECHNISCHES GEBIET

Die vorliegende Erfindung betrifft ein Verfahren zur Bestimmung des Sehvermögens eines Benutzers, sowie ein Datenverarbeitungsprogramm zur Durchführung des Verfahrens auf einem tragbaren elektronischen Gerät. Insbesondere geht es um ein Verfahren, welches es dem Benutzer selbstständig und regelmässig ohne grossen Aufwand ermöglicht, über eine längere Zeit und z.B. im Rahmen einer medizinischen Behandlung diese Funktionen zu überprüfen, um dann rechtzeitig medizinische Hilfe in Anspruch nehmen zu können, wenn diese erforderlich wird.

### STAND DER TECHNIK

Aus dem Stand der Technik sind diverse Verfahren bekannt, um die Sehfähigkeit zu überprüfen respektive zu überwachen. So beschreibt beispielsweise die US 4798456 ein Verfahren, mit welchem quantitativ Metamorphopsie ausgemessen werden kann, indem der betroffenen Person unterschiedliche Bilder vorgelegt werden, und die Person anschliessend über die Wahrnehmung befragt wird.

Aus der WO2010132304 ist beispielsweise ein Handsehkrafttester und ein Verfahren zur Selbstprüfung der Sicht eines Benutzers unter Verwendung einer solchen Vorrichtung bekannt. Das Verfahren gewährleistet, dass in einer Anzeige des Handsehkrafttesters innerhalb einer akzeptablen Distanz zum Auge des Benutzers Abweichungen vom zulässigen Abstand ausgeglichen werden. Im Rahmen des Verfahrens werden verschiedene Formen, entweder dynamisch oder statisch, auf der Anzeige dem Benutzer dargestellt. Das Verfahren ermöglicht auch die Eingabe von Reaktionen des Benutzers auf die verschiedenen Formen, die wiederum angezeigt werden. Ergebnisse des Selbsttests werden aus den vom Benutzer erzeugten Reaktionen bestimmt. Dargestellt werden dabei beispielsweise Kreise neben deformierten Kreisen, und der Benutzer muss die deformierten Kreise identifizieren.

Aus der US 4 798 456 ist ein Verfahren zur Messung der Sehfähigkeit eines Benutzers bekannt, bei welchem zwei beabstandete aber in ihrer Position fixierte Punkte vorgegeben werden, und der Patient wird aufgefordert, einen dritten Punkt entlang der Verbindungsachse der beiden äusseren fixierten Punkte in die Mitte zwischen diese beiden fixierten Punkte zu verschieben.

Aus der US 2005/122477 ist ein Verfahren bekannt, bei welchem ein Testmuster vorgegeben wird, und der Benutzer aufgefordert ist, an diesem Testmuster bestimmte Änderungen vorzunehmen, und aus der entsprechenden Verhaltensrückmeldung des Benutzers werden Schlüsse gezogen über das Sehverhalten des Benutzers. Ähnliches ist aus der US 2012/050685 bekannt.

### DARSTELLUNG DER ERFINDUNG

Es ist entsprechend Aufgabe der vorliegenden Erfindung, ein verbessertes Verfahren zur Bestimmung des Sehvermögens (wobei dieser Begriff das Gesichtsfeld miteinschliessen soll) eines Benutzers zur Verfügung zu stellen, welches ohne medizinische Unterstützung zuverlässig und fehlerfrei durchgeführt werden kann.

Diese Aufgabe wird gelöst durch ein Verfahren nach Anspruch 1 respektive ein Datenverarbeitungsprogramm nach Anspruch 15 zur Durchführung eines solchen Verfahrens oder durch ein mobiles elektronisches Gerät mit einem solchen Verfahren, wobei das mobile elektronische Gerät nicht Teil der Erfindung ist.

Der vorgeschlagene Test dient dazu, die Fähigkeit des Sehsystems zu messen. Indirekt kann über die Fähigkeit des Sehsystems aber auch ein Rückschluss gezogen werden über generelle Fähigkeiten des Benutzers, beispielsweise die Reaktionsfähigkeit, die Belastbarkeit oder den Entspannungszustand. Entsprechend kann indirekt auf den generellen physiologischen Zustand, beispielsweise auch auf den Alkoholisierungsgrad oder generell auf den Zustand unter dem Einfluss von Drogen oder Medikamenten, unter dem Einfluss einer auch gegebenenfalls nicht direkt mit den Augen und der Sehfähigkeit im Zusammenhang stehenden Krankheit oder eines psychischen Zustands oder einer Kombination dieser Aspekte, ein Rückschluss gezogen werden. Mit dem Verfahren wird die Fähigkeit überprüft, Punkte in ihrer räumlichen Beziehung zueinander zu erfassen. Diese Qualität des Sehens ist als sogenannter Vernier Visus bekannt. Diese Sehfähigkeit basiert auf der Sehleistung der Netzhaut und höherer Strukturen in der visuellen Signalverarbeitung. Die Genauigkeit dieser Sehleistung kann durch verschiedene Alterungsprozesse oder Krankheitsprozesse beeinflusst werden. Zu diesen Prozessen gehören die Altersabhängige Maculadegeneration (AMD), die diabetische Retinopathie, Gefässverschlüsse jeglicher Art in der Netzhaut und der Sehbahn, das Maculaödem jeglicher Genese (z.B. postoperativ, diabetisch, entzündlich), Erkrankungen der Netzhaut, die zu Verlagerung der Fotorezeptoren führen, wie beispielsweise die epiretinale Fibroplasie, das vitreofoveale Traktionssyndrom und das Maculaloch, sowie Erkrankungen des Sehnerven wie zum Beispiel das Glaukom. Die Lage der dargebotenen Punkte kann so gewählt werden, dass diese gezielt auf die betroffenen Netzhautstellen gelegt werden. Die Wahl der Lage der Punkte kann anhand von morphologischen Untersuchungen wie zum Beispiel der optischen Kohärenzuntersuchung (OCT) oder auf Basis der Ergebnisse vorgängiger Untersuchungen mit dem hier beschriebenen Verfahren (adaptives oder lernendes Verfahren) getroffen werden. Zusätzlich kann bei wiederholter Anwendung durch die vergleichende Analyse der Präzision und der Geschwindigkeit der Eingaben und Antworten durch die Testperson auch der Einfluss tagesbedingter Leistungsschwankungen oder von krankheitsbedingten Veränderungen des Gehirns auf die Art oder Geschwindigkeit der Bildwahrnehmung oder der Einfluss exogener Substanzen wie Alkohol oder andere sedative und halluzinogene Substanzen auf die Reaktionsfähigkeit des Gehirns gemessen werden. Durch die dynamische Komponente der Vernier-Visusprüfung können netzhautbedingte und nicht netzhautbedingte Veränderungen oder Schwankungen der Messresultate erfasst und die Detektionssensibilität des Systems für Funktionsänderungen der Netzhaut erhöht werden.

Konkret betrifft die vorliegende Erfindung ein Verfahren zur Bestimmung des Sehvermögens, bei welchem der Benutzer, eine Testserie von mehreren nacheinander durchgeführten individuellen Testvorgängen absolviert. Dabei werden jeweils im Rahmen eines individuellen Testvorgangs auf einem Display eines elektronischen Geräts zwei beabstandete, innerhalb eines individuellen Testvorgangs ortsfeste Aussenmarken, und eine dazwischen, aber nicht auf einer Verbindungsgerade der Aussenmarken liegende Ausrichtungsmarke dargestellt. Der Benutzer ist nun aufgefordert, durch Betätigung des elektronischen Geräts die Ausrichtungsmarke auf dem Display so weit senkrecht zur Verbindungsgerade der Aussenmarken zu verschieben, bis nach Wahrnehmung des Benutzers die Ausrichtungsmarke auf der Verbindungsgerade zwischen den Aussenmarken liegt. Auf diese Weise fokussiert der Benutzer sein Sehen auf die Ausrichtungsmarke, muss aber diese relativ zu den Aussenmarken positionieren.

Anschliessend nach erfolgter Verschiebung der Ausrichtungsmarke durch den Benutzer, registriert und analysiert das elektronische Gerät wenigstens einen der folgenden registrierten Parameter:
den effektiven Abstand der Ausrichtungsmarke von der Verbindungsgerade nach Verschiebung der Ausrichtungsmarke durch den Benutzer;
die Zeit, die der Benutzer für die Verschiebung der Ausrichtungsmarke benötigt hat;
die Anzahl Richtungsänderungen bei der Verschiebung der Ausrichtungsmarke durch den Benutzer.

Typischerweise wird der individuelle Testvorgang beendet, indem der Benutzer eine entsprechende Eingabe macht, beispielsweise indem er auf ein Feld "weiter" auf dem Display einwirkt.

Dabei werden wenigstens zwei oder wenigstens drei individuelle Testvorgänge durchgeführt, bei welchen die Aussenmarken entlang der gleichen Hauptachse oder vorher bestimmter Zusatzachsen angeordnet sind, und wobei wenigstens zwei verschiedene Hauptachsen im Rahmen einer Testserie ausgemessen werden. Anschliessend wird als Mass zur Ermittlung des Sehvermögens aus den individuellen Testvorgängen der Testserie wenigstens einer der registrierten Parameter oder, bei der Messung von mehreren Parametern, vorzugsweise eine Kombination der registrierten Parameter, verwendet. Konventionelle Messverfahren zur Bestimmung des Vernier-Visus wie z.B. das Amsler-Gitter haben den Nachteil, dass die geprüfte Person einen bestimmten Punkt fixieren muss, aber dazu aufgefordert wird, Seheindrücke zu beurteilen, die ausserhalb des fixierten Punktes liegen. Beim hier beschriebenen Verfahren wird gewissermassen die Situation umgedreht, ohne dass der Benutzer dies bemerkt, was die Zuverlässigkeit und die Einfachheit der Messung wesentlich vereinfacht.

Im Zusammenhang mit der vorliegenden Erfindung gelten folgende Definitionen:
*Elektronisches Gerät:* darunter ist generell ein Gerät zu verstehen, welches dazu in der Lage ist, einem Benutzer ein Bild zur Verfügung zu stellen. Es handelt sich um Geräte für die ein- oder beidäugige Darstellung von zwei oder dreidimensionalen Bildern. Darunter fallen entsprechend mobile Geräte (z.B. Tablets und Smartphones) und Personal Computer (z.B. Desktop-Computer und Laptops), aber auch Projektoren, Head-mounted Devices, interaktive Brillen, 3-D-Darstellungsgeräte, holographische Darstellungsgeräte, (Gross-)Bildschirme, Datenbrillen, Datenhelme, Hologramm-Laser etc.

*Display:* darunter ist nicht nur ein Display im klassischen Sinne zu verstehen, sondern generell eine Vorrichtung zur Darstellung von 2 oder 3-dimensionalen Bildern für ein oder beide Augen. Der Begriff schliesst mit anderen Worten nicht nur klassische Displays ein, sondern auch auf eine Fläche projizierte Darstellungen, sowie die Darstellungsmittel wie sie beispielsweise in Datenbrillen, oder in holographischen Darstellungen eingesetzt werden. Bevorzugt werden Displays die berührungsempfindlich sind.

*Aussenmarken:* als Aussenmarken werden die ausserhalb des unmittelbaren visuellen Aufmerksamkeitsbereichs des Benutzers angeordneten Marken bezeichnet, wobei deren Form im Prinzip nicht weiter spezifiziert ist, solange sie dazu in der Lage sind, eine dazwischen gedachte Verbindungsgerade für den Benutzer visuell vorstellbar zu machen. Die Aussenmarken, die beispielsweise als Punkte oder Striche oder auch Kreise oder Dreieck ausgebildet sein können, können ergänzt oder verstärkt werden durch zusätzliche Hilfsmittel wie beispielsweise Kreise oder Schattierungen etc.

*Ausrichtungsmarke:* als Ausrichtungsmarke wird das Darstellungselement zwischen den Aussenmarken bezeichnet. Auch dieses Element der Aussenmarke ist hinsichtlich Form im Prinzip nicht weiter spezifiziert, solange es ermöglicht, diese Aussenmarke in möglichst nachvollziehbarer Weise auf der gedachten Verbindungsgerade zwischen den Aussenmarken zu positionieren. Ausrichtungsmarke und Aussenmarken können auf einem hellen Hintergrund gewissermassen positiv dargestellt werden, sie können aber auch auf einem dunklen Hintergrund gewissermassen negativ dargestellt werden. Ebenfalls denkbar ist eine gemischte Darstellung, d.h. auf einem mittleren Hintergrund werden die Aussenmarken als dunkler Marken dargestellt und die Ausrichtungsmarke als hellere Markierungen, oder umgekehrt.

*Abstand:* unter dem Abstand der Ausrichtungsmarke ist der Abstand der Ausrichtungsmarke von der gedachten Verbindungsgerade zwischen den beiden Aussenmarken zu verstehen. Der Abstand ist entsprechend gleich null, wenn die Ausrichtungsmarke genau auf der gedachten Verbindungsgerade zwischen den beiden Aussenmarken liegt. Der Abstand verändert sich im Rahmen eines individuellen Testvorgangs in der Regel. Anfangs wird zwischen den Aussenmarken eine nicht auf einer Verbindungsgerade der Aussenmarken liegende Ausrichtungsmarke dargestellt, d.h. der Abstand ist am Anfang ungleich Null, die Grösse des Initial-Abstandes wird bevorzugtermassen zufällig vorgegeben, wobei für diesen Anfangswert typischerweise ein absoluter Minimalwert und ein absoluter Maximalwert für diese zufällige Vorgabe angesetzt wird. Im Laufe des individuellen Testvorgangs verschiebt der Benutzer die Ausrichtungsmarke entlang einer Richtung senkrecht zur gedachten Verbindungsgerade der Aussenmarke, er kann die Ausrichtungsmarke gar nicht entlang anderer Achsen verschieben, und am Ende des individuellen Testvorgangs resultiert ein Abstand, der dem effektiven Abstand der Ausrichtungsmarke von der Verbindungsgerade nach Verschiebung der Ausrichtungsmarke entspricht.

*Versatz:* Der Versatz der Ausrichtungsmarke ist die Position der Ausrichtungsmarke relativ zur Distanz zwischen den beiden Aussenmarken. Betrachtet man die gedachte Verbindungsgerade zwischen den beiden Ausrichtungsmarken als x-Achse und die senkrecht dazu stehende Achse als y-Achse, so ist entsprechend der Versatz die x-Koordinate der Ausrichtungsmarke und der oben definierte Abstand der Ausrichtungsmarke ist die y-Koordinate der Ausrichtungsmarke.

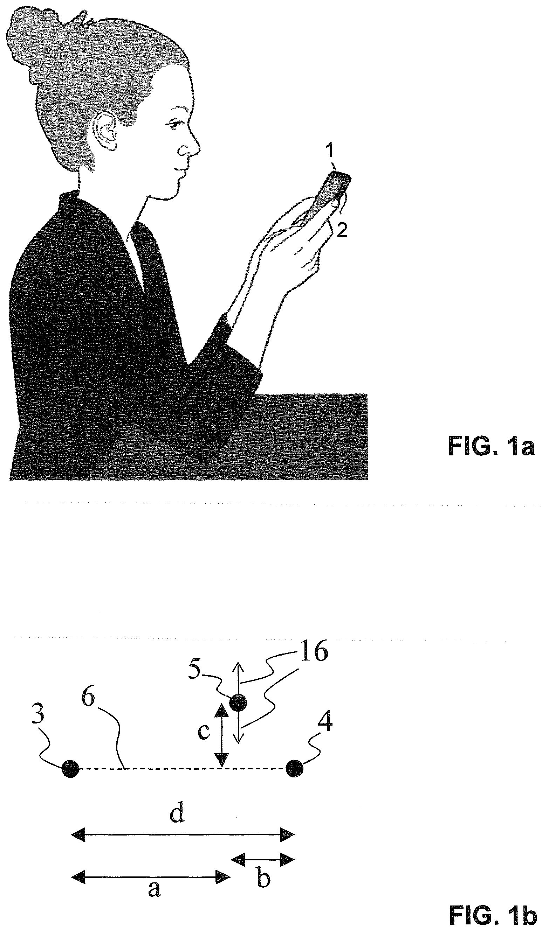

Grafisch sind die Verhältnisse in Figur 1b dargestellt für die Situation einer horizontalen Hauptachse. Der oben definierte Abstand entspricht der Grösse c in dieser Figur, die Distanz zwischen den Aussenmarken 3 und 4 entspricht d, und die Grösse a entspricht dem Versatz. Die Ausrichtungsmarke 5 kann nur entlang der senkrecht zur Verbindungsgerade 6 verlaufenden Verschiebungsrichtung 16 verschoben werden.

*Hauptachsen:* unter den Hauptachsen werden die vertikale (vertikale Hauptachse) und die horizontale Achse (horizontale Hauptachse) verstanden, wie sie dem Benutzer bei der bestimmungsgemässen Benutzung des Displays des entsprechenden elektronischen Geräts erscheinen. Die Richtung der entsprechenden Hauptachse entspricht der gedachten Verbindungsgerade zwischen den beiden Aussenmarken.

*Diagonale Achsen oder Zusatzachsen:* unter diesen zusätzlichen Achsen sind Achsen zu verstehen, die unter einem Winkel zwischen den Hauptachsen angeordnet sind. Diese Zusatzachsen können unter einem Winkel von 45° zur vertikalen respektive horizontalen Hauptachse angeordnet sein, und damit die konventionell als diagonale Achsen bezeichneten Achsen sein. Unter diagonalen Achsen nach der vorliegenden Erfindung sind aber generell Achsen zu verstehen, diese eine für diese Anwendungen genügende WinkelAbweichung von der vertikalen und der horizontalen Hauptachse aufweisen. Typischerweise schliessen diese diagonalen Hauptachsen bevorzugtermassen unabhängig voneinander einen Winkel mit der horizontalen respektive vertikalen Hauptachse von 10-80°, vorzugsweise im Bereich von 20-70°, insbesondere bevorzugtermassen im Bereich von 30-60° ein. Ganz bevorzugt schliessen diese diagonalen Hauptachsen mit der horizontalen respektive vertikalen Hauptachse einen Winkel im Bereich von 40-50°, ganz bevorzugt einen Winkel im Bereich von 45° oder genau bei 45° ein. Gibt es zwei derartige zusätzliche Achsen, so können diese senkrecht zueinander stehen, d.h. sie schliessen untereinander einen Winkel von 90° ein, es ist aber auch möglich, dass die diagonalen Hauptachsen untereinander einen Winkel im Bereich von 20-160° einschliessen, so beispielsweise im Bereich von 60-120°. Bevorzugtermassen schliessen die diagonalen Hauptachsen untereinander einen Winkel von 80-100° ein, insbesondere einen Winkel im Bereich von 90° oder genau von 90°.

*Betätigung des elektronischen Geräts:* diese Betätigung, entweder zur Überführung in eine nächste individuelle Messung oder zur Verschiebung der Ausrichtungsmarke, kann entweder direkt auf dem Display erfolgen (beispielsweise bei Verwendung eines berührungsempfindlichen Displays), oder aber es ist möglich, eine zusätzliche Input-Schnittstelle vorzusehen. Über diese können beispielsweise auch Bewegungsinformation des Benutzers (Kopfnicken oder andere Körperteil-Bewegungen) registriert werden, zur Registrierung kann entweder eine Zentraleinheit oder eine spezielle Vorrichtung zur Betätigung vorgesehen werden, beispielsweise kann bei einer Datenbrille die Bewegung der Ausrichtungsmarke durch Bewegung des Kopfes, durch Spracheingabe, oder durch entsprechende manuelle Eingabe an der Datenbrille gesteuert werden.

Da das Sehsystem darauf trainiert ist, immer den Punkt zu fixieren, der beurteilt werden soll, ist es für den Geprüften im Rahmen der klassischen Bestimmung des Vernier Visus über das Amsler-Gitter fast unmöglich diese Aufgabe zu erfüllen ohne die Fixation vom geforderten Fixationspunkt abzuwenden. Die Untersuchung wird dadurch aber wertlos. Der Vorteil des hier beschriebenen Testverfahrens liegt darin, dass der Untersuchte den Punkt fixieren muss, den er verschieben muss. Dies entspricht dem normalen physiologischen Sehvorgang, indem man seine visuelle Aufmerksamkeit dem Punkt widmen kann, der manipuliert werden muss. Da der Untersuchte den zu manipulierenden Punkt im Verhältnis zu den ebenfalls dargestellten zwei Aussenpunkten ausrichtet, sind die effektiv getesteten Punkte diese beiden Aussenpunkte. Dieses Messverfahren ermöglicht also die Messung von Punkten ausserhalb des zentralen Fixierpunktes, ohne dass der Untersuchte sich bewusst auf Punkte ausserhalb des Fixierpunktes konzentrieren muss. Durch eine dynamische Anpassung der Testabbildung auf die effektiven Testbedingungen (Abstand Auge zum Bild oder Rotation des Kopfes oder des Bildschirmes) wird die Qualität und Zuverlässigkeit der Messungen erhöht. Durch die zusätzliche Registrierung und Auswertung der benötigten zeitlichen Dauer der Teilaufgaben, bis der Nutzer die Ausrichtungsmarke subjektiv optimal auf der Verdingungsgeraden positioniert hat, sowie durch die Registrierung und Auswertung der Anzahl und Grösse der Steuerbewegungen pro Teilaufgaben wird die statische Prüfung der Vernier-Funktion um eine dynamische Komponente erweitert, wodurch die Sensibilitätsschwelle für die Entdeckung degenerativer oder auch krankheitsbedingter Veränderungen gesenkt werden kann. Das Verfahren erlaubt zudem eine kontinuierliche Anpassung des Tests und der Darstellung an die Fähigkeiten und Bedürfnisse jedes individuellen Auges eines Nutzers.

Gemäss der vorliegenden Erfindung ist das Verfahren dadurch gekennzeichnet, dass im Rahmen einer Testserie wenigstens zwei oder wenigstens drei Testvorgänge entlang der vertikalen Hauptachse und zwei oder wenigstens drei Testvorgänge entlang der horizontalen Hauptachse durchgeführt werden. Zusätzlich werden jeweils wenigstens zwei oder wenigstens drei Testvorgänge entlang der beiden diagonalen Hauptachsen durchgeführt.

Unter diesen diagonalen Hauptachsen ist nicht streng zu verstehen, dass diese schrägen Hauptachsen unter einem Winkel von 45° zur vertikalen respektive horizontalen Hauptachse stehen müssen. Wesentlich ist, dass diese diagonalen Hauptachsen eine für diese Anwendungen genügende Abweichung von der vertikalen und der horizontalen Hauptachse aufweisen. Typischerweise schliessen diese diagonalen Hauptachsen bevorzugtermassen unabhängig voneinander einen Winkel mit der horizontalen respektive vertikalen Hauptachse von 10-80°, vorzugsweise im Bereich von 20-70°, insbesondere bevorzugtermassen im Bereich von 30-60° ein. Ganz bevorzugt schliessen diese diagonalen Hauptachsen mit der horizontalen respektive vertikalen Hauptachse einen Winkel im Bereich von 40-50°, ganz bevorzugt einen Winkel im Bereich von 45° oder genau bei 45° ein.

Bevorzugtermassen sind die beiden diagonalen Hauptachsen senkrecht zueinander, d.h. sie schliessen untereinander einen Winkel von 90° ein, es ist aber auch möglich, dass die diagonalen Hauptachsen untereinander einen Winkel im Bereich von 20-160° einschliessen, vorzugsweise im Bereich von 60-120°. Bevorzugtermassen schliessen die diagonalen Hauptachsen untereinander einen Winkel von 80-100° ein, insbesondere einen Winkel im Bereich von 90° oder genau von 90°.

Die Anordnung der Testpunkte auf verschiedenen Achsen ermöglicht die Testung verschiedener, genau definierter Netzhautstellen. Dies ermöglicht degenerative, von Geburt an vorhandene oder krankhafte Prozesse bestimmten Arealen und Stellen der Netzhaut zuzuordnen. Tatsächlich kann der ganze Bereich der Macula damit detailliert untersucht werden. Der Messbereich erstreckt sich über mindestens einen Kreis von einem Radius von 10 Grad um den Fixationspunkt herum. Da gemäss vorgeschlagenem Verfahren die gemessenen Punkte sehr genau und reproduzierbar gemessen werden können, ist diese Zuordnung sehr genau im Gegensatz zu andern Verfahren z.B. dem Amsler-Gitter, das diese Zuordnung nur ungenau zulässt. Konkret ist es möglich durch entsprechende geschickte Führung der Messung respektive der gewählten Ausgangslagen entlang der verschiedenen Hauptachsen durch gezielte Datenauswertung die schwächeren Bereiche auf der Netzhaut für jedes Auge effektiv zu bestimmen. Es können so Areale durch die unterschiedlichen Messungen entlang verschiedener Achsen und mit verschiedenen Abständen der Ausrichtungsmarke von den Aussenmarken auf der Netzhaut identifiziert werden, wobei diese Areale typischerweise Kreissegmente sind oder, infolge der verschiedenen Abstände der Ausrichtungsmarke von der Aussenmarke, sogar Kreisringsegmente. So kann gewissermassen ein Mapping auf einem Polargitter erfolgen, wobei die verschiedenen Gitterflächen individuell beurteilt werden können.

Es können wie oben dargelegt verschiedene Parameter als Mass zur Ermittlung des Sehvermögens des Benutzers eingesetzt werden. Nach einer weiteren bevorzugten Ausführungsform wird bei Registrierung des Parameters des effektiven Abstandes der Ausrichtungsmarke von der Verbindungsgerade nach Verschiebung der Ausrichtungsmarke durch den Benutzer als Mass zur Ermittlung des Sehvermögens der Mittelwert und/oder die Standardabweichung des Betrags des effektiven Abstandes der Testvorgänge entlang der gleichen Hauptachse verwendet.

Wird zusätzlich oder alternativ eine Registrierung des Parameters der Zeit vorgenommen, die der Benutzer für die Verschiebung der Ausrichtungsmarke benötigt hat, und diese als Mass für die Ermittlung des Sehvermögens gewertet, dann kann der Mittelwert und/oder die Standardabweichung der Gesamtzeit zwischen dem Erscheinen der Marken und dem Quittieren des Endes des individuellen Testvorgangs verwendet werden. Es ist auch möglich, wenn als Ende des individuellen Testvorgangs beispielsweise automatisch, bei fehlender Eingabe innerhalb einer bestimmten Anzahl von Sekunden, zum nächsten Messvorgang fortgeschritten wird, als Ende den Zeitpunkt zu nehmen, zu welchem die letzte Manipulation stattgefunden hat.

Wird zusätzlich oder alternativ als Parameter eine Registrierung der Anzahl Richtungsänderungen bei der Verschiebung der Ausrichtungsmarke durch den Benutzer registriert, so kann der Mittelwert und/oder die Standardabweichung der Anzahl Richtungsänderungen bei den individuellen Testvorgängen verwendet werden oder die Summe der Richtungsänderungen entlang einer bestimmten Hauptachse. Unter diesem Parameter können auch zusätzlich noch die Amplitude und/oder die Frequenz von solchen Richtungsänderungen registriert und ausgewertet werden.

Es können neben den hier spezifisch genannten Parametern auch noch weitere Parameter registriert werden und in die Datenauswertung zur Bestimmung des Sehvermögens einfliessen. So beispielsweise Informationen, die über die auf den Benutzer gerichtete Kamera aufgenommen werden, so beispielsweise Informationen über das Auge, die Aktivität des Auges während der Messung etc. Ebenfalls einfliessen kann die Position des Gerätes relativ zum Benutzer, und die Bewegung des Gerätes (über entsprechende Gyro-Elemente/Sensoren), sowie Informationen über die Helligkeit der Umgebung, die Tageszeit, die Zeit seit der letzten Untersuchung etc.

Da der Vernier-Visus abhängig von verschiedenen physiologischen Einflussfaktoren wie z.B. Tageszeit, Lerneffekt und Ausrichtung der geprüften Achse variiert, werden diese Einflussfaktoren durch zusätzliche Analyse der dynamischen Testparameter wie benötigte Zeitdauer für die Teilaufgaben und Anzahl und Grösse der benötigten Steuer- und Korrekturbewegungen ergänzt. Dies ermöglicht die bessere Unterscheidung zwischen sehfähigkeitsbedingten und tagesformbedingten oder durch Lemeffekte hervorgerufene Veränderung der Messresultate. Dadurch können, soweit das Verfahren dazu eingesetzt wird, netzhautbedingte Einflüsse auf das Sehvermögen auszumessen, nichtnetzhautbedingte Einflüsse auf die Messgenauigkeit ausgefiltert oder richtig gewichtet werden, und damit die Sensitivität des Verfahrens für die Bestimmung sehfähigkeitsbedingter Veränderungen erhöht werden. Auf der anderen Seite können gerade diese weiteren Parameter, wenn das Verfahren beispielsweise generell die Wahrnehmungsfähigkeit des Benutzers auszumessen beabsichtigt (beispielsweise im Sinne eines Alkoholtests), als relevante zusätzliche Parameter in der Auswertung berücksichtigt werden. Diese weiteren Parameter können also sowohl zur weiteren Verfeinerung der Messresultate eingesetzt werden, als auch zur Verhinderung der Verfälschung der Messresultate.

Gemäss der Erfindung ist das Verfahren dadurch gekennzeichnet, dass Testvorgänge entlang der gleichen Hauptachse mit jeweils unterschiedlichem Versatz der Ausrichtungsmarke entlang der Verbindungsgerade begonnen werden, wobei vorzugsweise entlang jeder Hauptachse zu Beginn des jeweiligen Testvorgangs ein Versatz von 1/3, 1/2 und 2/3 der Distanz zwischen den beiden Aussenmarken vorgegeben wird. Die Anzahl der individuellen Testvorgänge und/oder der jeweilige Versatz kann dabei bevorzugtermassen auf Basis von vorherigen

Untersuchungsresultaten oder vorangegangenen morphologischen Untersuchungen individuell angepasst werden. Durch diese unterschiedliche Relativposition der Ausrichtungsmarke zu den Aussenmarken ist es möglich, nicht nur Kreissektoren auf der Netzhaut respektive des Sichtfeldes auszumessen, sondern Kreisringsektoren.

Die Testmethode lässt die Untersuchung eines sehr grossen Areales der Netzhaut zu. Degenerative, krankhafte oder andere Prozesse beschränken sich aber oft auf eng umschriebene Gebiete der Netzhaut. Durch den zu Beginn gewählten Versatz der Punkte lassen sich betroffene Gebiete im Sinne einer Suchfunktion rasch auffinden und im weiteren Verlauf der Messung durch Wahl der Lage der Messpunkte näher eingrenzen. Konkret ist es nämlich möglich, zukünftige Testserien in Abhängigkeit von Messresultaten vorhergehender Testserien spezifisch anzupassen, respektive auf die entscheidenden Messungen zu reduzieren. Durch die Reduktion der Anzahl von individuellen Testmessungen kann auch deren Genauigkeit wesentlich erhöht werden, da Ermüdungserscheinungen der Benutzer einen geringeren Einfluss haben sollten.

Gemäss einer weiteren bevorzugten Ausführungsform kann es sich bei den Aussenmarken und der Ausrichtungsmarke um Striche oder Kreise mit einer Länge respektive Durchmesser im Bereich von 1-10 mm, vorzugsweise im Bereich von 2-4 mm handeln. Im Falle von Strichen kann es sich um solche mit einer Strichdicke im Bereich von 0.5-5 mm, vorzugsweise im Bereich von 1-2.5 mm handeln. Dabei sind alle Striche bevorzugtermassen während des ganzen Testvorgangs parallel zur auszumessenden Hauptachse ausgerichtet. Die Grösse der Aussenmarken und der Ausrichtungsmarke können gleich sein, und sie können in ihrer Grösse dem Sehvermögen der Testperson angepasst sein.

Gemäss einer weiteren bevorzugten Ausführungsform wird auf Basis von vorherigen Testserien und/oder vorherigen individuellen Testvorgängen und/oder vorangegangenen morphologischen Untersuchungen wenigstens einer der folgenden Einstellungsparameter individuell angepasst: die Grösse der Marken; der Kontrast auf dem Display; die Farbwahl auf dem Display; gegebenenfalls zusätzlich vorhandene Fixierhilfen, wobei diese Individualisierung vorzugsweise für jedes spezifische Auge des Benutzers erfolgt.

Der Benutzer führt bevorzugtermassen die Testserie mit nur einem Auge durch und/oder das elektronische Gerät gewährleistet, dass die auf dem Display dargestellte Information nur von einem Auge wahrgenommen werden kann.

Also kann das Verfahren sowohl für jedes Auge und jeden Benutzer individualisiert werden. Entsprechend kann, in Abhängigkeit von Messresultaten, die Form und Grösse der Marken, der Kontrast, oder allfällige Fixierhilfen für jedes Auge individualisiert und für jede Eingabe während dem Messvorgang dargestellt werden.

Je kleiner die Messmarken gewählt werden umso präziser lässt sich die Sehqualität Vernier Visus bestimmen. Die Erkennung der Testmarken hängt allerdings von der Sehqualität bzw. Sehschärfe ab. Je schlechter die Sehschärfe, umso grösser müssen sowohl die Ausrichtungsmarke als auch die Aussenmarken sein, damit sie von der untersuchten Person erkannt werden können. Beim vorliegenden Test kann ggf. deshalb die Grösse, Art und Kontrast der Testmarke der Sehschärfe der zu prüfenden Person angepasst werden. Dadurch kann insbesondere bei Testpersonen mit stark verminderter Sehschärfe noch zuverlässig die Sehqualität Vernier-Visus getestet werden. Dies unterscheidet diesen Test deutlich von anderen Verfahren zur Prüfung des Vernier Visus wie z.B. Amsler-Gitter, wo die Testmuster konstante Grösse aufweisen. Ist die Sehschärfe des zu untersuchenden Auges so weit reduziert, dass die Testperson Mühe hat, die Fixation auf die Aussenmarken konstant aufrecht zu halten, können zusätzliche Fixierhilfen wie z.B. eine ausserhalb der Testmarken liegende Ringdarstellung um das ganze Testfeld herum, eingeblendet werden. Ein weiteres bevorzugtes Verfahren ist dadurch gekennzeichnet, dass der Benutzer das Verschieben der Ausrichtungsmarke durch eine direkte oder indirekte Interaktion mit dem elektronischen Gerät kontrolliert, wobei diese Interaktion durch ein berührungsempfindliches Display, Körperbewegungen, insbesondere der Hand oder des Kopfes, oder durch Sprache, oder eine spezifische Eingabeschnittstelle wie beispielsweise ein manuelles Eingabegerät oder aber auch über ein über Bluetooth angekoppeltes Eingabegerät (beispielsweise wenn das Display über eine Projektion erfolgt) oder eine Kombination davon, erfolgen kann, wobei, wenn es sich beim Display um ein berührungsempfindliches Display handelt, der Benutzer entweder die Ausrichtungsmarke direkt durch berühren und verschieben der Ausrichtungsmarke auf dem Display ausrichtet, oder unter Zuhilfenahme eines oder mehrerer Verschiebungsknöpfe, die auf dem Display abgebildet sind. Die Art, Grösse, Anzahl, Richtung und der Zeitbedarf der Interaktion des Nutzers mit den Eingabehilfen zur Steuerung der Testmarke in den Zielbereich kann ebenfalls gemessen und für die Verbesserung der Mess-Sensitivität wie auch zur Beurteilung der Tagesform der Nutzer oder der des Lerneffektes genutzt werden.

Da oft ältere Personen an Veränderungen der Netzhaut leiden, kann die Interaktion mit dem Messgerät erschwert sein. Durch die vielfältigen gemäss der vorliegenden Erfindung möglichen Möglichkeiten der Interaktion lassen sich diese Schwierigkeiten umgehen. Die Interaktionen, mit denen der Test gesteuert wird, entsprechen alltagstypischen Interaktionsmustern, indem mit Gesten oder Kopfbewegungen wie Kopfnicken oder Kopfschütteln eine Interaktion erfolgen kann.

Wie bereits dargelegt ist es möglich, durch die Messung der Zeit, bis eine korrekte Positionierung der Testmarke erreicht wird, zusätzlich zur eigentlichen Vernier-Visus Messung indirekt eine Veränderung der Sehfunktion festzustellen. Bereits sehr feine Verschlechterungen der Sehfunktion führen zu einem erhöhten Zeitbedarf für die korrekte Positionierung der Prüfmarke. Hinweise auf eine Verschlechterung der Sehfunktion in einem gestimmten Netzhautareal bestehen dann, wenn die Zeit zur Lösung der Teilaufgabe auf einer bestimmten Achse oder einem Punkt im Vergleich zu früheren Untersuchungen länger wird, oder wenn die Differenz zwischen den langsam und den schnell gelösten Teilaufgaben im Vergleich zu früheren Untersuchungen zunimmt. Wird auf einer oder mehreren Prüfachsen ein erhöhter Zeitbedarf zum Lösen der Aufgabe festgestellt (benötigte Zeit liegt beispielsweise ausserhalb von zwei Standardabweichung der für diese Testpunkte in früheren Untersuchungen benötigte Zeit), wird das Gerät ein verkürztes Intervall bis zur nächsten Testung (z.B. 3 statt 7 Tage) vorschlagen. Wird eine über mehrere Messtage progrediente Verlangsamung festgestellt, werden zusätzliche Massnahmen, wie beispielsweise einer Aufforderung, einen Optiker oder Arzt aufzusuchen, oder weitere Massnahmen empfohlen. Stellt das Verfahren fest, dass der Zeitbedarf für alle Aufgaben zu- oder abnimmt, ist das ein Hinweis auf eine allgemein bessere oder schlechtere Sehfunktion, eine schwankende Tagesform des Nutzers oder auf einen Lerneffekt. Diese Information kann verwendet werden, um bei einem eindeutigen Trend die Grösse der Testmarken anzupassen, oder die Resultate bezüglich der Verwendung in der individuellen Datenbank (Baseline für das gewohnte Testresultat einer Achse oder eines Areals) unterschiedlich zu gewichten. Dadurch kann vermieden werden, dass Untersuchungsresultate von Testprüfungen an allgemein schlechten Tagen des Nutzers oder der Effekt der Lernkurve den statistischen Referenzwert für eine erwartete Leistung des Nutzers auf einer Achse falsch tief werden lässt. Dadurch bleibt die Sensitivität des Verfahrens trotz Lernkurve oder geänderten Test-Darstellungen unverändert hoch.

Das Verfahren bietet gegenüber den bisherigen Messverfahren mit Analyse rein statischer Messgrössen der Vernier-Sehfunktion die Möglichkeit durch die Messung einer dynamischen Komponente die Sensibilität für die Entdeckung einer Veränderung der Vernier-Sehfunktion wesentlich zu erhöhen und auch Effekte wie Tagesform, Lernkurve oder geänderte Darstellung zu berücksichtigen und zu kompensieren.

Eine wie oben bereits dargelegt ebenfalls mögliche Messung der notwendigen Anzahl Korrekturbewegungen sowie der Grösse und /oder Amplitude der überschiessenden Korrekturbewegungen bis zur definitiven Positionierung der Prüfmarke kann registriert werden. Eine vermehrte Anzahl Korrekturbewegungen oder eine Zunahme der Amplitude der Korrekturbewegungen auf einer Achse oder mehreren Achsen im Vergleich zu den in vorhergehenden Untersuchungszyklen festgestellten Werten ist ein Hinweis auf eine lokale Verschlechterung der Vernier-Sehfunktion. Hinweise auf eine Verschlechterung der Sehfunktion in einem bestimmten Netzhautareal bestehen dann, wenn die Anzahl Korrekturbewegungen zur Lösung der Teilaufgabe auf einer bestimmten Achse oder einem Punkt im Vergleich zu früheren Untersuchungen zunimmt, oder wenn die Differenz zwischen den am besten und den am schlechtesten gelösten Teilaufgaben im Vergleich zu früheren Untersuchungen zunimmt. Analog zu einer gemessenen Verschlechterung der zeitlichen Komponente der Vernier-Sehfunktionsprüfung wird bei einer Verschlechterung der Anzahl oder der Amplitude der dynamischen Komponente der Vernier-Sehfunktionsprüfung das Verfahren einen reduzierten Intervall bis zur nächsten Prüfung oder zusätzliche Massnahmen empfehlen.

Der Vorteil dieser zweiten Komponente der dynamischen Prüfung der Vernier-Sehfunktion ermöglicht ebenfalls eine erhöhte Sensitivität der Vernier Sehfunktionsprüfung für die frühe Feststellung sehr feiner Verschlechterungen der Sehfunktion. Zudem können Einflussgrössen wie die Tagesform oder Lerneffekte von sehfähigkeitsbedingten Veränderungen besser unterschieden werden, da Tagesform und Lerneffekt die Anzahl und Grösse der Korrekturbewegungen für alle Teilaufgaben, Achsen und Netzhautareale beeinflusst, während krankheitsbedingte Veränderungen vornehmlich im Bereich bereits erkrankter Netzhautareale oder nur einzelner Areale mit schlechter Funktion auftreten. Eine Messung der notwendigen Anzahl Korrekturbewegungen sowie der Grösse (Amplitude) der überschiessenden Korrekturbewegungen bis zur definitiven Positionierung der Prüfmarke kann unter Berücksichtigung der statischen Resultate der Vernier-Sehfunktionsprüfung (Abstand der Marke von der Achse) erfolgen. Wird eine starke und generelle Zunahme der benötigen Anzahl und Amplitude der erfolgten Korrekturbewegungen (dynamische Komponente) auf allen Achsen ohne Verschlechterung der statischen Testkomponente (Erhöhung des effektiven Abstandes der Ausrichtungsmarke von der Verbindungsgerade) festgestellt, dann ist dies ein Hinweis auf eine Veränderung der Tagesform der Testperson oder ein Effekt der Lernkurve oder ein Effekt der geänderten Testdarstellung oder verursacht durch eine allgemeine Verlangsamung der zentralnervösen Leistung und Steuerung der Koordination z.Bsp. als Folge von sedativen, halluzinogenen oder berauschenden Substanzen wie Alkohol. In diesem Fall werden die Resultate dieser Messung der Vernier-Sehfunktion für die Festlegung der individuellen Referenzwerte der langfristigen Sehfunktion der Testperson nicht oder nur in reduzierter Form berücksichtigt. Dadurch kann vermieden werden, dass Untersuchungsresultate von Testprüfungen an allgemein schlechten Tagen des Nutzers oder der Effekt der Lernkurve den statistischen Referenzwert für eine erwartete Leistung des Nutzers auf einer Achse falsch tief werden lässt. Dadurch bleibt die Sensitivität des Verfahrens trotz Lernkurve oder geänderten Test-Darstellungen unverändert hoch. Bei einer plötzlich auftretenden, erheblichen Abweichung von der üblichen Tagesform (mehr als 2 Standardabweichungen) wird das Verfahren der Testperson empfehlen, Massnahmen zu Ergreifen oder temporär Tätigkeiten zu unterlassen (z.B. Führen von Fahrzeugen und Nutzung gefährlicher Maschinen).

Der Vorteil der kombinierten dynamischen und statischen Analyse der Vernier-Funktion ist, dass neben einer visuellen Komponente auch die Reaktionsfähigkeit der Testperson gemessen werden kann. Dadurch können kurzfristige Verschlechterungen als Folge der fluktuierenden Tagesform der Testpersonen (Müdigkeit, Tageszeit, sedierende Substanzen) gegenüber netzhautbedingten Verschlechterungen teilweise oder sogar vollständig abgegrenzt werden. Dadurch können durch die aktuelle Tagesform bedingte Verschlechterungen gegenüber der rein netzhautbedingten Verschlechterung der Sehfunktion bezüglich der langfristigen Analyse statistisch untergewichtet werden. Durch das Herausfiltern des durch die Tagesform bedingten Rauschens der Vernier-Sehfunktion wird die Sensibilität der Prüfung für die rein netzhautbedingten Veränderungen der Vernier-Sehfunktion nochmals erhöht.

Eine weitere bevorzugte Ausführungsform des vorgeschlagenen Verfahrens ist dadurch gekennzeichnet, dass auf dem Display in Abhängigkeit des ermittelten Sehvermögens eine qualitative Auswertung oder eine quantitative Auswertung ausgegeben wird, oder ein Hinweis, dass medizinische Hilfe aufgesucht werden soll.

Gemäss einer anderen bevorzugten Ausführungsform ist die Anordnung der Aussenmarken und der Ausrichtungsmarke für die verschiedenen individuellen Testvorgänge individuell aufgrund der statistischen Auswertung der vorangegangenen Messungen angepasst.

Im Gegensatz zu anderen Messverfahren lässt der vorliegende Test eine quantitative Messung des Vernier Visus einzelner Netzhautareale zu. Die Messresultate der einzelnen Testpunkte sind für sich genommen wenig aussagekräftig im Hinblick auf die Dynamik eines krankhaften Vorganges und für einen Benutzer des Testes nicht interpretierbar. Durch statistische Methoden unter Berücksichtigung vorangegangener Messungen oder anderer in derselben Untersuchung erfasster Messpunkte lässt sich jedoch die Dynamik eines krankhaften oder degenerativen Prozesses ableiten. Nur diese Information und nicht die Resultate einzelner Messpunkte sind für den Benutzer von Belang und werden dem Nutzer direkt zur Verfügung gestellt.

Die in einer Testserie ermittelten Daten, gegebenenfalls in Kombination mit Informationen über den Benutzer, in personalisierter oder anonymisierter Form, können zudem gemäss einer weiteren bevorzugten Ausführungsform an eine zentrale Stelle übermittelt werden, vorzugsweise über eine Internet- oder Mobiltelefon-Verbindung, und an dieser zentralen Stelle die Daten gespeichert, weiterverarbeitet, und/oder zur Information respektive Weiterbearbeitung an eine medizinische Betreuung weitergeleitet wird.

Das elektronische Gerät kann zusätzlich über Sensoren (einschliesslich eine auf den Benutzer gerichtete Kamera, Bewegungssensoren/Beschleunigungssensoren, Orientierungssensoren, optische oder thermische Sensoren, Schall- oder Geruchssensoren), und damit das Verhalten des Benutzers, insbesondere den Abstand des Benutzers zum Display, und/oder den Augenabstand, und/oder ob der Testvorgang ein- oder beidäugig durchgeführt wird, und in ersterem Fall, welches Auge dabei geschlossen ist, und/oder die Rotation des Displays um die Sehachse und/oder die Orthogonalität der Sehachse zur Fläche des Displays bestimmen. Diese Information kann man in die Datenanalyse einfliessen lassen und/oder über das Display oder akustische Signale oder Stimmausgabe dem Benutzer einen Hinweis übermitteln, die entsprechende Grösse auf den korrekten Wert einzustellen. Zusätzlich oder alternativ ist es möglich, diese Information zur optimalen Durchführung und Aufzeichnung der Messung einzusetzen. So kann vorteilhafter Weise auf Basis dieser Informationen die auf dem Display erfolgte Darstellung von Aussenmarken und Ausrichtungsmarke angepasst werden, vorzugsweise sogar dynamisch innerhalb eines Testvorgangs oder zwischen den Testvorgängen einer Serie anzupassen. Die Kamera oder andere Sensoren des elektronischen (Geräts) können zusätzlich zur eindeutigen Identifikation einer Testperson oder von deren Verhalten während des Messvorgangs oder auch vor respektive nach dem Messvorgang verwendet werden (z.B. via Gesichtserkennung, Fingerabdruck oder Stimmerkennung). Auch kann die Lage oder Grösse der Testmarken auf dem Display insbesondere abhängig von der gemessenen effektiven Distanz und der Drehung des Displays dynamisch während des Testvorgangs so angepasst werden, dass die Projektion der Messpunkte auf die Netzhaut konstant bleibt.

Messverfahren zur Bestimmung des Verniervisus können nur reproduzierbare und quantifizierbare Resultate liefern, wenn sichergestellt ist, dass repetitiv gleiche Netzhautstellen gemessen werden können. Das vorliegende Verfahren passt sich diesbezüglich mittels Sensoren dynamisch dem Benutzer an.

Beim elektronischen Gerät, das nicht Teil der Erfindung ist, handelt es sich vorzugsweise um ein tragbares Gerät, insbesondere ein Personal Digital Assistant (PDA), Smartphone, Mobiltelefon, Tablet, Laptop, Smartwatch, Datenbrille oder Head-Mounted Display. Dabei kann das Verfahren auf einer zwei oder dreidimensionalen Abbildung beruhen. Der Testung der Vernier Sehfunktion kann einäugig oder beidäugig erfolgen. Die beidäugige Testung führt zu einem reduzierten Zeitbedarf für die Testung beider Augen. Die Zeiteinsparung kann bei Bedarf für die Messung an zusätzlichen Arealen oder zusätzliche Messungen an kritischen Arealen genutzt werden.

Die Resultate von verschiedenen nacheinander durchgeführten Testserien, vorzugsweise von an unterschiedlichen Tagen durchgeführten Testserien, können in ihrer Entwicklung vom elektronischen Gerät relativ zueinander gewertet werden und die Entwicklung des Sehvermögens kann ermittelt werden, und vorzugsweise bei Erreichen eines Schwellenwerts einer aus den Testserien berechneten Grösse eine Warnung an den Benutzer ausgegeben werden und/oder über eine Schnittstelle an eine zentrale Stelle zur Kontaktaufnahme mit dem Benutzer übermittelt werden.

Zur Erfassung und Überwachung krankhafter Prozesse ist die Überprüfung der Dynamik des Sehvermögens entscheidend. Einzelne Messungen für sich genommen sind nur von begrenzter Aussagekraft. Die Bewertung innerhalb des Verlaufs erhöht die Aussagekraft deutlich und lässt auch andere, nicht krankheitsbedingte, nicht interessierende Schwankungen der Sehfunktion ausfiltern. Durch das Ausfiltern der nicht krankheitsbedingten Schwankungen der Sehfunktion kann die Genauigkeit und Sensibilität des Verfahrens für die Feststellung krankheitsbedingter Veränderungen gegenüber bisherigen Verfahren zur Bestimmung der Vernier-Sehfunktion erheblich erhöht werden. Gemäss einer weiteren bevorzugten Ausführungsform des hier vorgeschlagenen Verfahrens kann das elektronische Gerät den Benutzer im Rahmen eines vorgegebenen Zeitplans auffordern, jeweils zu einem bestimmten Zeitpunkt eine Testserie durchzuführen, wobei vorzugsweise in Abhängigkeit des Resultats von einer vorhergehenden Testserie und insbesondere je nach Änderungen der Resultate der unmittelbar vorhergehenden Testserie gegenüber den durch multiple vorhergehende Testserien ermittelten Basiswert, dem Benutzer eine erneute Testserie im Rahmen eines festen zeitlichen Schemas, beispielsweise alle 3-14 Tage, oder vorzugsweise alle 7 Tage, oder bei einer festgestellten Verschlechterung der Resultate bereits nach einem individuell verkürzten Zeitintervall empfohlen wird.

Die Durchführung von Sehtests kann für die Untersuchten eine grosse Belastung darstellen und somit dazu führen, dass die Tests nicht mehr oder nicht mehr mit der genügenden und angemessenen Aufmerksamkeit durchgeführt werden. Da viele Netzhauterkrankungen chronischer Natur sind, ist eine Testung über grosse Zeiträume nötig. Die Frequenz der Testvorgänge erfolgt beim vorliegenden Test deshalb nicht nach einem fixen Zeitschema, sondern wird aufgrund vorheriger Messresultate und anderer Faktoren individuell angepasst. Zusätzlich kann auch spezifisch die Sequenz der individuellen Messungen ganz gezielt auf die Problemzonen fokussiert werden, namentlich das nur jene Hauptachsen ausgemessen werden, welche tatsächlich für den entsprechenden Benutzer relevant sind. Die Steuerung der Zeitintervalle kann auch individuell nur für dasjenige Auge mit einer Veränderung der Resultate erfolgen (z.B. wird bei einer Verschlechterung des linken Auges nur das empfohlene Untersuchungsintervall des linken Auges verkürzt). Durch die individuelle Steuerung der Zeitintervalle bis zur nächsten Prüfung, kann die zeitliche Belastung des Nutzers minimiert werden. Zudem kann an einem Auge mit einer Verschlechterung zusätzliche Messungen oder Prüfachsen für eine verbesserte Messgenauigkeit oder Testsensibilität hinzugefügt werden, während an einem stabilen Auge nur noch eine kurze Basisprüfung durchgeführt werden muss. Zudem wird die Testperson zur Testung jeweils aufgefordert.

Wie bereits erwähnt, können gemäss einer weiteren bevorzugten Ausführungsform die Messdaten des elektronischen Gerätes an einen zentralen Rechner gesendet werden und dort, unter Verwendung von Algorithmen ausgewertet werden. Mögliche Algorithmen sind beispielsweise die Ermittlung von i) der Varianzzunahme der Messpunktabstände zu einer Vergleichsmessung ii) der Berücksichtigung von Patientencharakteristika, wie insbesondere Alter, Geschlecht, augenärztliche Befunde wie Sehstärke, Augen- und Systemerkrankungen; iii) subjektiv erlebter Schwierigkeitsgrad der Testdurchführung, Testdauer, Anzahl und Amplitude der Steuerung und Korrektur der Testmarke und dem Verlauf der Testergebnisse, sowie allfälligen therapeutischen Massnahmen.

Die Resultate solcher Analysen können als eine personalisierte Rückmeldung, vorzugsweise für jedes Auge individualisiert, aufbereitet und dem Benutzer zur Verfügung gestellt werden und die Resultate können dazu verwendet werden, die Messanordnung zu individualisieren, wobei diese Individualisierung in einer Änderung der Messachsen, der Messpunkte, des Messintervalls und der Beurteilung der Messwerte bestehen kann. Die Testung erfolgt vorzugsweise mit einem abgedeckten Auge. Der Test kann auch mit beiden Augen gleichzeitig durchgeführt werden, wobei dann je nach Testergebnissen zur Durchführung mit einem zugedeckten Auge aufgefordert werden kann.

Des Weiteren betrifft die vorliegende Offenbarung ein tragbares elektronisches Gerät, das nicht Teil der Erfindung ist, zur Durchführung eines Verfahrens wie es oben dargelegt wurde mit einem Display und einer Datenauswertungseinheit, wobei es sich vorzugsweise beim Display um ein berührungsempfindliches Display handelt, und wobei weiterhin vorzugsweise das elektronische Gerät zusätzlich über eine zum Benutzer gerichtete Kamera verfügt. Die Darstellung der Testaufgaben kann je nach Fähigkeit des verwendeten Gerätes zwei- oder dreidimensional erfolgen.

Zu guter letzt betrifft die vorliegende Erfindung ein Datenverarbeitungsprogramm zur Durchführung eines Verfahrens wie es oben dargelegt wurde auf einem Gerät wie oben dargestellt, vorzugsweise in Form einer mobilen Applikation (App).

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: in a) eine Benutzerin des vorgeschlagenen Verfahrens, die ein mobiles Gerät im vorgeschlagenen Abstand von ca. 30 cm vor ihren Augen hält, und in b) eine graphische Darstellung der relativen Anordnung von entlang einer horizontalen Hauptachse angeordneten Aussenmarken und einer Ausrichtungsmarke und der entsprechenden Distanzen und Abstände sowie Bezeichnungen für die Situation, wo der Versatz ca. 2/3 beträgt;

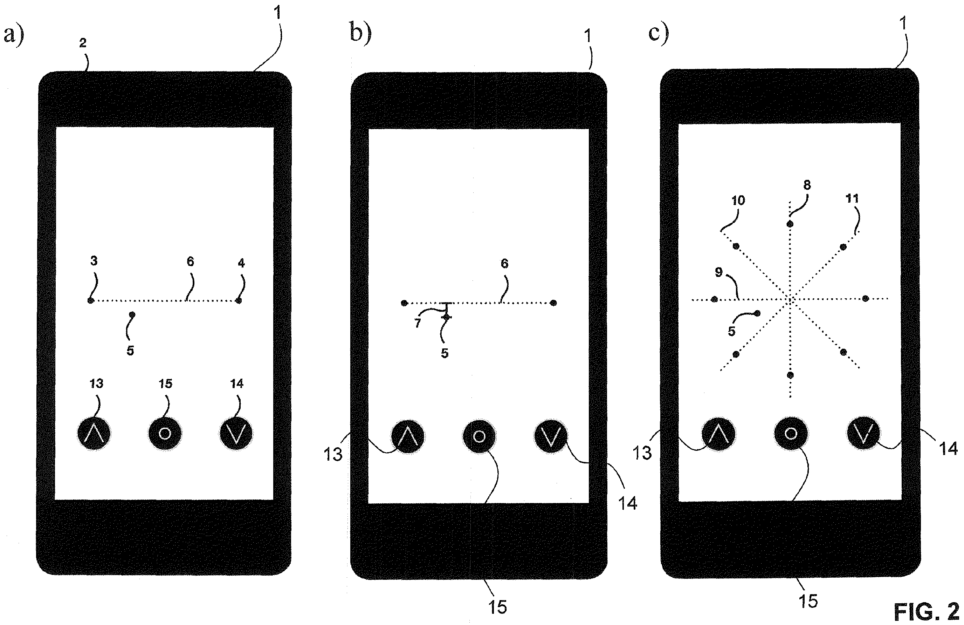

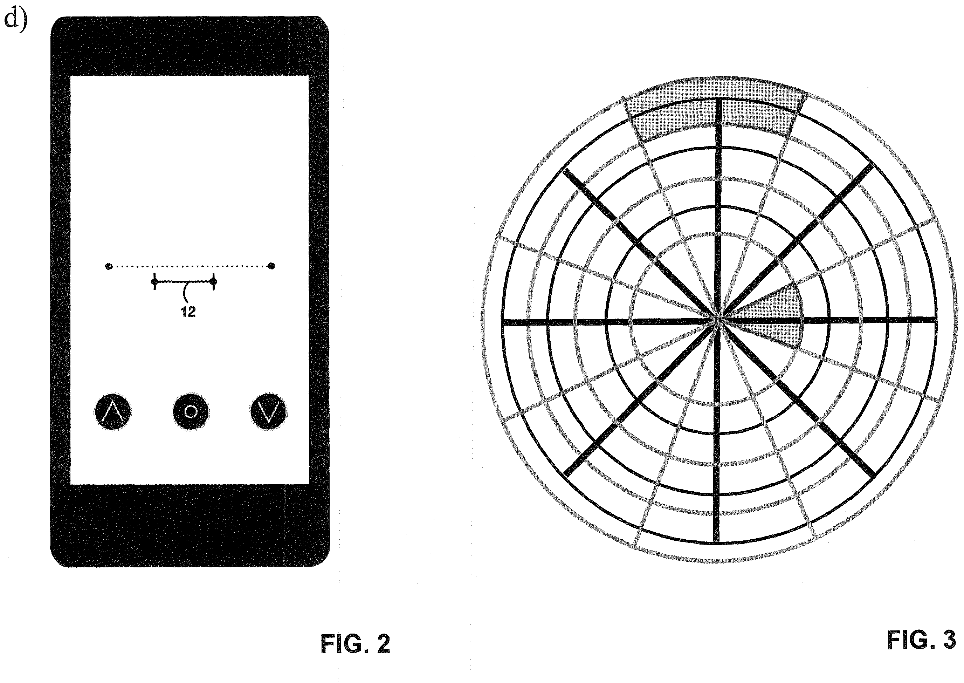

- Fig. 2: die unterschiedlichen Darstellungen im vorgeschlagenen Verfahren, wobei in a) die Ausgangssituation eines individuellen Testvorgangs dargestellt wird, bei welchem die Aussenmarken horizontal angeordnet sind; in b) die Lage der Ausrichtungsmarke nach Manipulation des Benutzers dargestellt ist, in c) die verschiedenen Hauptachsen gleichzeitig dargestellt sind, und in d) der Bereich für den Versatz für die Situation, wo die Aussenmarken horizontal angeordnet sind, schematisch angegeben ist; und
- Fig. 3: mögliche topographische Resultate der Messung in einem Polargitter dargestellt, wobei in schwarz die Messachsen dargestellt sind: diese bilden die Mittelinien von Netzhautsektoren, deren Begrenzungen grau dargestellt sind; die grauen Flächen markieren betroffene Netzhautsektoren; anhand der Auswertung betroffener Sektoren kann das System anhand der Testung in den Randbereichen selbstlernend angepasst, präzisiert und optimiert werden.

### BESCHREIBUNG BEVORZUGTER AUSFÜHRUNGSFORMEN

In Figur 1 ist eine Benutzerin dargestellt, die ein Smartphone 2 vor Augen hält, das über ein berührungsempfindliches Display 1 verfügt. Auf diesem Smartphone 2 ist eine Software in Form einer mobilen Applikation (App) geladen, welches das vorgeschlagene Verfahren implementiert. Dabei ist darauf zu achten, dass die Sehachse im Wesentlichen senkrecht zur Oberfläche des Displays 1 ausgerichtet ist, und dass die lange Achse des Displays ungefähr senkrecht zur Verbindungslinie der Augen ausgerichtet ist, auf jeden Fall sollte die Drehung des Displays relativ zum Gesicht des Benutzers innerhalb einer Testserie nicht verändert werden.

Damit die Benutzerin auf eine korrekte Ausrichtung des Smartphones hingewiesen wird ist es möglich, die Kamera und gegebenenfalls weitere Sensoren (z.B. Gyroskop und Accelerometer) des Smartphones ebenfalls in die Software einzubinden, und nach Starten der Software zunächst über die Kamera respektive andere Sensoren festzustellen, zum Beispiel über eine entsprechende Gesichtserkennung eines durch die Kamera aufgenommenen Bildes des Benutzers, ob eine korrekte Relativposition von Display zum Benutzer eingenommen ist, und gegebenenfalls durch entsprechende Warnhinweise oder sogar durch konkrete Änderungshinweise (beispielsweise "das Display befindet sich 2 cm zu nahe bei ihren Augen, bitte weiter entfernt halten", oder "der Monitor ist zur Kopfrichtung verdreht - bitte im Uhrzeigersinn 5° drehen", oder durch Einblendung einer Hilfsachse), die entweder bildlich, schriftlich oder auch akustisch ausgegeben werden können, eine optimale Positionierung zu veranlassen. Die falsche Ausrichtung des Displays in Bezug auf die Augen oder Abweichungen der Prüfungsdistanz kann ggf. durch dynamische Änderung der Lage der Punkte auf dem Display während des Testablaufs kontinuierlich und automatisch kompensiert werden, oder es ist möglich, die registrierten Parameter in Abhängigkeit solcher Abweichungen zu korrigieren, sodass die Abweichungen zur korrekten Haltung auf das Messresultat keinen Einfluss haben. Zudem können über die Gesichtserkennung die Person identifiziert, die Messseite (linkes bzw. rechtes Auge), die einäugige Messung und andere, die Qualität der Messung beeinflussende Parameter erhoben werden und die Darstellung oder der Algorithmus des Testablaufes den individuellen Sehfähigkeiten jeweils des linken oder rechten Auges einer Testperson angepasst oder korrigiert werden.

Unter Verwendung derartiger Sensoren (Bewegungssensoren/Beschleunigungssensoren, Orientierungssensoren, etc.) ist es möglich, das generelle Verhalten der Person, die die Tests durchführt, zu überwachen, davon ausgehend, dass die Person das Messgerät stets auf sich trägt. Das Messgerät kann mit anderen Worten gleichzeitig auch als Activity Tracker verwendet werden, und die daraus ermittelten Daten können in die Auswertung der hier beschriebenen Testvorgänge eingebaut werden. So kann beispielsweise aus einer an sich gleichbleibenden generellen Aktivität des Nutzers vor und nach dem Messvorgang daraus geschlossen werden, dass obwohl vielleicht die Messergebnisse etwas schlechter ausgefallen sind, die Aktivität hingegen gleich geblieben ist, offenbar die schlechteren Messergebnisse keine lebenserhebliche Beeinträchtigung des Sehverhaltens bedeuten. Die Aktivität des Nutzers kann auch über separate am Körper befestigte Sensoren (z.B. Armband, ActivityTracker) erfasst und direkt oder indirekt an das Gerät und/oder an eine zentrale Stelle übermittelt werden, die dann diese Aktivität in der Auswertung der vom Gerät an die zentrale Stelle übermittelten Daten berücksichtigt.

Die Ausgangslage bei einem ersten individuellen Testvorgang ist in Figur 2a dargestellt. In diesem Fall ist die Hauptachse 6 horizontal angeordnet, d.h., die beiden Aussenmarken 3 und 4 sind ungefähr auf halber Höhe auf dem Display am rechten respektive linken Rand dargestellt, und die Verbindungsgerade 6 verläuft horizontal.

Dabei ist zu beachten, dass im effektiven Test die hier in Figur 2 jeweils gepunktet dargestellte Verbindungsgerade 6 selbstverständlich nicht sichtbar gemacht ist, sondern eben gerade weggelassen ist, damit der sich auf die Ausrichtungsmarke 5 konzentrierende Benutzer gezwungen ist, sich gewissermassen durch nicht im Fokus liegende Bereiche definiert durch die beiden Aussenmarken 3 und 4 zu orientieren und damit die genau auszumessenden Bereiche zu benutzen.

Als Ausgangssituation wird die gewissermassen zwischen den beiden Aussenmarken 3 und 4 angeordnete Ausrichtungsmarke 5 in diesem Fall wesentlich nach unten versetzt und etwas links vom Zentrum zwischen den beiden Aussenmarken 3 und 4 angeordnet.

Auf dem Display 1 gibt es nun im unteren Bereich drei berührungsempfindliche Bereiche. Ganz links aussen ist ein erster Bereich 13 angegeben, welcher bei Berühren mit dem Finger die Ausrichtungsmarke 5 nach oben, d.h. in diesem Fall näher zur Verbindungsgerade 6 und senkrecht zu dieser verschiebt. Ganz rechts aussen ist ein zweiter Bereich 14 angegeben, mit welchem die Ausrichtungsmarke in die entgegengesetzte Richtung ebenfalls senkrecht zur Verbindungsgerade verschoben werden kann. Diese beiden Bereiche 13 und 14 sind immer dazu vorgesehen, dass die Ausrichtungsmarke nur in einer respektive der entgegengesetzten Richtung senkrecht zur Verbindungsgerade verschoben werden kann. Die auf den Bereichen 13 respektive 14 dargestellten Pfeile drehen sich entsprechend, damit der Benutzer erkennen kann, welche Funktion respektive welche Richtung relativ zur Verbindungsgerade welchem Knopf zugewiesen ist.

In der Mitte befindet sich zudem ein weiterer berührungsempfindlicher Bereich, mit welchem der individuelle Testvorgang abgeschlossen werden kann und zum nächsten fortgeschritten werden kann.

Die verschiedenen berührungsempfindlichen Bereiche 13-15 können selbstverständlich auch in anderen Bereichen des Gesamtdisplays angeordnet sein.

Weiterhin ist zu bemerken, dass anstelle der Bereiche 13 respektive 14 auch so vorgegangen werden kann, dass direkt durch Fingerauflegen auf das berührungsempfindliche Display und ein entsprechendes Verschieben des Fingers die Positionierung der Ausrichtungsmarke 5 vorgenommen werden kann.

Ausgehend von der in Figur 2a dargestellten Ausgangssituation wird nun der Benutzer durch das Betätigen der Bereiche 13 respektive 14 die Ausrichtungsmarke so weit verschieben, bis er subjektiv das Gefühl hat, sie liege auf der Verbindungsgerade 6 (die eben im effektiven Versuch nicht dargestellt wird, obwohl in Figur 2 zur Erleichterung der Beschreibung dargestellt). Ist der Benutzer der Meinung, dass die Ausrichtungsmarke 5 auf der Verbindungsgerade 6 liegt, beispielsweise in der Position wie in Figur 2b dargestellt, kann er durch den zentralen Bereich 15 zur nächsten individuellen Testvorgang fortschreiten. Wenn er dies tut, wird von der Software der Abstand 7 der Ausrichtungsmarke 5 von der Verbindungsgerade 6 ausgelesen und gespeichert. Dieser Abstand 7 wird, wie weiter unten beschrieben werden wird, für die Auswertung eingesetzt. Zusätzlich kann auch die benötigte Zeit bis die Testperson die Marke subjektiv korrekt ausgerichtet hat, sowie die Anzahl und Amplitude benötigter Richtungskorrekturen bei der Verschiebung der Marke registriert und anschliessend zur Auswertung analysiert werden. Es werden nun in einer für den Benutzer zufällig scheinenden Weise verschiedene solche individuellen Testvorgänge durchgeführt, wobei jeweils wenigstens drei Testvorgänge entlang einer Hauptachse (vergleiche Beschreibung von Figur 2 c) durchgeführt werden. In Figur 2c sind die verschiedenen dabei ausgemessenen Hauptachsen dargestellt. Einerseits die horizontale Hauptachse 9, wie sie auch im individuellen Testvorgang gemäss den Figuren 2a und b illustriert ist, dann die vertikale Hauptachse 8, sowie eine erste diagonale Hauptachse 10 und eine zweite diagonale Hauptachse 11.

Werden sämtliche Hauptachsen ausgemessen und auf jeder Hauptachse drei Messungen vorgenommen, ergibt dies 12 Messungen, was bisweilen als beschwerlich empfunden werden kann. Um die Anzahl auch tatsächlich sinnvoller Messungen möglichst gering zu halten oder durch schwerpunktmässige Messungen am Rand der erkrankten Netzhautareale feine Veränderungen besser und früher darzustellen, ist es möglich, indem jeweils die gemessenen Daten einer Testserie an einen zentralen Rechner übermittelt werden, die individuellen Testvorgänge mit früheren Testresultaten zu vergleichen und für spätere Messserien anzupassen auf das, was auch tatsächlich vorher als problematisch festgestellt wurde. Hat ein Benutzer beispielsweise eine Schwäche, die sich durch die Messungen auf der zweiten diagonalen Hauptachse manifestiert, nicht aber Probleme auf der ersten diagonalen Hauptachse, so kann die erste diagonale Hauptachse nicht bei jeder Testserie ausgemessen werden oder nur noch einzelne Messungen und nicht mehr mehrere pro Achse. Die Analyse der Resultate und Anpassung des Testablaufes erfolgt vorzugsweise individuell für jedes getestete Auge.

Wie oben bereits dargelegt, werden die Ausrichtungsmarken bei den individuellen Testvorgängen in unterschiedlicher Position relativ zu den beiden Aussenmarken in der

Ausgangssituation vorgelegt. Der dabei sinnvollerweise mögliche Versatz 12 ist in Figur 2d illustriert. Typischerweise bewegt sich dieser Versatz zwischen entweder im Zentrum oder zwischen 1/3 und 2/3 der Distanz der beiden Aussenmarken 3, 4. Zur Präzisierung eines Messresultates, zum Erstellen eines Netzhaut-Mappings (Figur 3), zur Ermittlung des Gesichtsfeldes oder als Reaktion auf ausgeprägte Seheinschränkungen eines Auges, kann der Testvorgang hinsichtlich der Form und Grösse der Aussenmarken, deren Abstände, des Kontrastes, allfälliger zusätzlicher Fixierhilfen oder anderen Massnahmen, wie beispielsweise Farbänderung für jedes Auge individualisiert dargestellt werden.

Zur Auswertung der eigentlichen Testvorgänge innerhalb der Testserie werden nun die Abstände 7, die die Ausrichtungsmarke 5 jeweils zum Zeitpunkt hat, wenn der Benutzer zum nächsten individuellen Testvorgang wechselt (vergleiche Figur 2b), gesammelt und statistisch ausgewertet. Dabei kann beispielsweise so vorgegangen werden, dass der Mittelwert des Absolutbetrags der Messungen entlang einer bestimmten Achse verwendet wird sowie deren Streuung. Anschliessend können Mittelwert und Streuung in Bezug auf jede der 4 Achsen als Messresultat ausgegeben werden. Weitere, während der Messung erhobene Parameter wie beispielsweise die Dauer des gesamten Untersuchs oder die Dauer bis zur Positionierung einzelner Punkte, sowie die Anzahl und Grösse oder Amplitude der Korrekturbewegungen beziehungsweise die Veränderungen dieser Parameter bei wiederholten Messungen können zur Verbesserung der Messung oder zur Senkung der Detektionsschwelle für krankhafte Veränderungen zusätzlich verwendet werden. Des Weiteren ist es möglich, dass entlang jeder Hauptachse die geschädigten Areale identifiziert werden und dort ein Fein-Mapping des entsprechenden Netzhautabschnittes vollzogen werden kann. Im Zusammenhang mit dem Netzhaut-Mapping können die Abstände der Aussenmarken für einzelne Achsen angepasst werden.

Das Messresultat kann entweder auf dem Smartphone selber autonom erzeugt werden, es kann aber auch so vorgegangen werden, dass die Rohinformation (einzelne Messpunkte) an einen zentralen Server übergeben wird, dort die Auswertung geschieht, insbesondere auch in Abhängigkeit bereits erfolgter Testserien, und eine Rückmeldung nach zentraler Auswertung erfolgt.

Es ist auch möglich bei einer Kommunikation mit einem zentralen Rechner, eine entsprechende Rückmeldung nicht nur dem Benutzer, sondern auch beispielsweise einem Optiker oder medizinischem Personal, zukommen zu lassen.

Die Rückmeldung kann einerseits in einer statistischen Auswertung mit Zahlen bestehen, andererseits aber auch in einer detaillierteren Form erfolgen, beispielsweise mit der Aufforderung, einen Optiker oder medizinisches Personal aufzusuchen, weitere Massnahmen zu treffen oder Tätigkeiten, wie beispielsweise das Führen von Fahrzeugen du die Bedienung von gefährlichen Maschinen zu unterlassen.

Möglich ist es beispielsweise, den Benutzer zunächst oder jeweils in definierten Intervallen aufzufordern, eine Initialisierungsmessserie durchzuführen, die gewissermassen eine Basislinie der individuellen Fähigkeiten bestimmt. Aus diesen Initialisierungsmessungen wird ein Variationsbereich, der für den entsprechenden Nutzer charakteristisch ist, bestimmt, und zwar vorzugsweise für jede ausgemessene Achse individuell. D.h. bei Messung der horizontalen und der vertikalen Achse sowie der beiden diagonalen Achsen werden in diesen Initialisierungsmessungen vier Variationsbereiche ermittelt, für jede Achse einer. Dies bedeutet, dass entlang jeder Achse die erhaltenen Messwerte in dieser Initialisierungsmessung für jede Achse individuell einer statistischen Analyse unterzogen werden, und dann ein individueller Variationsbereich für die jeweilige Achse festgelegt wird. Dies geschieht vorzugsweise so, dass als Variationsbereich die doppelte Standardabweichung des Abstandes der Ausrichtungsmarke von der Verbindungsgerade eingesetzt wird.

Eine für den Benutzer nützliche direkte und hilfreiche Rückmeldung über das Display kann dann unabhängig von der weiteren statistischen Auswertung der Messungen im Hintergrund so ausgestaltet werden, dass, wenn alle 4 Achsen im jeweiligen Durchführen der Testserie ausgemessen werden, für jeden individuellen Testvorgang, bei welchem der Nutzer die Ausrichtungsmarke innerhalb des definierten Variationsbereichs (eben z.B. innerhalb des Bereichs +/- Standardabweichung wie bestimmt aus der Initialisierungsmessserie, wobei diese Standardabweichung ausgehend von Werten ermittelt in einer Initialisierungsmessserie im Laufe einer regelmässigen Fortführung der Testserien über einen längeren Zeitraum sukzessive angepasst werden kann) ausrichtet, ein Punkt vergeben wird. Werden pro Achse 3 Messungen durchgeführt entlang der 4 Achsen, ergibt sich so eine maximale pro Testserie von 12 Punkten. Nach Abschluss der Testserie kann dann über das Display die Anzahl erreichter Punkte ausgegeben werden, und mit einer zusätzlichen optischen Rückmeldung hinterlegt werden. So kann beispielsweise die Abweichung von der Maximalpunktzahl als Mass für die Qualität der Testserie angegeben werden, und bei Erreichen von 11 oder 12 Punkten ein Wert von 0 respektive 1 ausgegeben werden grün hinterlegt, im Sinne von alles in Ordnung, kein weiterer Handlungsbedarf, bei Erreichen von 9 oder 10 Punkten ein Wert von 2 respektive 3 ausgegeben werden, orange hinterlegt, im Sinne von Achtung, die Entwicklung ist nicht vorteilhaft, und bei Erreichen von weniger als 9 Punkten der entsprechende Differenzwert, jeweils rot hinterlegt im Sinne von Achtung Handlungsbedarf, bitte den Arzt kontaktieren. Die entsprechende Skala respektive bei welcher Punktzahl welche Warnung, optisch oder ergänzt durch zusätzliche Nachrichten, auch gegebenenfalls über den Audioausgang, ausgegeben wird, kann ebenfalls individuell eingestellt werden je nach medizinischer Situation, Alter, und Präferenz des Nutzers.

Der Test wird vorzugsweise nur mit einem Auge durchgeführt. Bei Geräten, bei denen beide Augen gleichzeitig die gleiche Bilddarstellung sehen können (z.B. Smartphone, Tablet-Computer und ähnliche) wird das andere Auge vorzugsweise abgedeckt oder zugedrückt. Kommt für die Abdeckung eine Augenklappe zum Einsatz, können auf dieser Muster, Zeichen oder maschinenlesbare Codes (z.B. QR-Code) angebracht sein, welche die Kamera erfassen kann und so Daten (z.B. zur Testperson) enthalten kann. Eine beidäugige Durchführung des Testes ist auch bei den oben erwähnten Geräten möglich, wobei die Verschlechterung der Sehfunktion vorerst nicht sicher einem bestimmten Auge zugeordnet werden kann. Wird bei der beidäugigen Untersuchung eine Veränderung der Sehfunktion bemerkt, kann der Untersuchte dazu aufgefordert werden, einäugige Tests durchzuführen. Welches Auge getestet werden muss, wird Anhand der Resultate der vorgängigen Untersuchungen bzw. deren Verlauf festgelegt. Bei Bildschirmen mit der Möglichkeit einer dreidimensionalen (räumlichen) Darstellung, kann durch Verwendung eines zusätzlichen Versatzes der Testmarke in der dritten Dimension die Testgenauigkeit einer beidäugigen Testung verbessert werden. Bei Durchführung des Testes mit Datenbrillen oder Geräten, die eine dreidimensionale oder virtuelle Realität darstellen oder jedem Auge ein unterschiedliches Bild präsentieren können, kann auch bei einer einäugigen Testung auf das Abdecken des kontralateralen Auges verzichtet werden.

**BEZUGSZEICHENLISTE**

| | | | |
|---|---|---|---|
| 1 | Display | 4 | Aussenmarke |
| 2 | elektronisches Gerät, Mobiltelefon, Smartphone, Smartwatch, Datenbrille | 5 | Ausrichtungsmarke |
| | | 6 | Verbindungsgerade |
| | | 7 | effektiver Abstand nach |
| 3 | Aussenmarke | | Manipulation des Benutzers |
| 8 | vertikale Hauptachse | | Bereich zu Eingabe des Endes des individuellen Testvorgangs |
| 9 | horizontale Hauptachse | | |
| 10 | erste diagonale Hauptachse | | |
| 11 | zweite diagonale Hauptachse | 16 | erlaubte Verschiebungs-richtung für die Aus-richtungsmarke |
| 12 | Versatz | | |
| 13 | berührungsempfindlicher Bereich zur Verschiebung der Ausrichtungsmarke in einer ersten Richtung senkrecht zur Verbindungsgerade | | |
| | | a | Versatz |
| | | b | komplementärer Versatz bezogen auf Gesamtdistanz d |
| | | c | Abstand der Ausrichtungsmarke |
| 14 | berührungsempfindlicher Bereich zur Verschiebung der Ausrichtungsmarke in einer zweiten Richtung senkrecht zur Verbindungsgerade | | |
| | | d | Gesamtdistanz zwischen den Aussenmarken |
| 15 | berührungsempfindlicher | | |

## Patentansprüche

1. Verfahren zur Bestimmung des Sehvermögens eines Benutzers, bei welchem dem Benutzer in Rahmen einer im Sinne einer Testserie geführten Mehrzahl von nacheinander durchgeführten individuellen Testvorgängen jeweils im Rahmen eines individuellen Testvorgangs auf einem Display (1) eines elektronischen Geräts (2) zwei beabstandete, innerhalb eines individuellen Testvorgangs ortsfeste Aussenmarken (3, 4), und eine dazwischen, aber nicht auf einer Verbindungsgerade (6) der Aussenmarken (3,4) liegende Ausrichtungsmarke (5) dargestellt werden,
und der Benutzer durch Betätigung des elektronischen Geräts (2) aufgefordert ist, die Ausrichtungsmarke (5) auf dem Display (1) soweit senkrecht zur Verbindungsgerade (6) der Aussenmarken (3, 4) zu verschieben, bis nach Wahrnehmung des Benutzers die Ausrichtungsmarke (5) auf der Verbindungsgerade (6) liegt,
und anschliessend nach erfolgter Verschiebung der Ausrichtungsmarke (5) durch den Benutzer das elektronische Gerät (2) wenigstens einen der folgenden Parameter registriert: den effektiven Abstand (7) der Ausrichtungsmarke (5) von der Verbindungsgerade (6) nach Verschiebung der Ausrichtungsmarke (5) durch den Benutzer; die Zeit, die der Benutzer für die Verschiebung der Ausrichtungsmarke (5) benötigt hat; die Anzahl Richtungsänderungen bei der Verschiebung der Ausrichtungsmarke (5) durch den Benutzer,
wobei wenigstens zwei oder wenigstens drei derartige individuelle Testvorgänge durchgeführt werden, bei welchen die Aussenmarken (3, 4) entlang der gleichen Hauptachse (8-11) oder vorher bestimmter Zusatzachsen angeordnet sind, und wobei wenigstens zwei verschiedene Hauptachsen im Rahmen der Testserie ausgemessen werden,
und wobei als Mass zur Ermittlung des Sehvermögens aus den individuellen Testvorgängen der Testserie der wenigstens eine registrierte Parameter oder, bei Messung von mehreren Parametern, eine Kombination der registrierten Parameter, verwendet wird,
**dadurch gekennzeichnet, dass** im Rahmen der Testserie wenigstens zwei der Testvorgänge entlang der vertikalen Hauptachse (8) des Displays (1) und zwei Testvorgänge entlang der horizontalen Hauptachse (9) des Displays (1) durchgeführt werden, und jeweils wenigstens zwei der Testvorgänge entlang wenigstens einer der beiden diagonalen Hauptachsen (10,11) durchgeführt werden und dass die Testvorgänge entlang der gleichen Hauptachse mit jeweils unterschiedlichem Versatz (12) der Ausrichtungsmarke (5) entlang der Verbindungsgerade (6) begonnen werden.

2. Verfahren nach Anspruch 1, **dadurch gekennzeichnet, dass** im Rahmen einer Testserie wenigstens drei Testvorgänge entlang der vertikalen Hauptachse (8) und wenigstens drei Testvorgänge entlang der horizontalen Hauptachse (9) durchgeführt werden, und jeweils wenigstens zwei oder wenigstens drei Testvorgänge entlang wenigstens einer der beiden diagonalen Hauptachsen (10,11) durchgeführt werden
wobei vorzugsweise bei entlang bestimmten Hauptachsen (8-11) registrierten Schwächen des Benutzers diese Hauptachsen besonders oder in der Folge nur noch im Wesentlichen ausschliesslich ausgemessen werden, und/oder wobei aus der entlang bestimmter Hauptachsen (8-11) ermittelten Sehfähigkeit auf die Fähigkeiten bestimmter Areale im Netzhautbereich Rückschlüsse gezogen werden, wobei diese Areale im Polargitter eines Polarkoordinatensystems definiert sind als Kreissektoren oder Kreisringsektoren.

3. Verfahren nach einem der vorhergehenden Ansprüche, **dadurch gekennzeichnet, dass**
bei Registrierung des Parameters des effektiven Abstandes (7) der Ausrichtungsmarke (5) von der Verbindungsgerade (6) nach Verschiebung der Ausrichtungsmarke (5) durch den Benutzer als Mass zur Ermittlung des Sehvermögens der Mittelwert und/oder die Standardabweichung des Betrags des effektiven Abstandes (7) der Testvorgänge entlang der gleichen Hauptachse verwendet wird;
oder bei Registrierung des Parameters der Zeit, die der Benutzer für die Verschiebung der Ausrichtungsmarke (5) benötigt hat als Mass für die Ermittlung des Sehvermögens der Mittelwert und/oder die Standardabweichung der Gesamtzeit zwischen dem Erscheinen der Marken (3-5) und dem Quittieren des Endes des individuellen Testvorgangs durch den Benutzer verwendet wird;
oder bei Registrierung des Parameters der Anzahl Richtungsänderungen bei der Verschiebung der Ausrichtungsmarke (5) durch den Benutzer der Mittelwert und/oder die Standardabweichung der Anzahl Richtungsänderungen bei den individuellen Testvorgängen verwendet wird oder die Summe der Richtungsänderungen entlang einer bestimmten Hauptachse, oder die Grösse und /oder Amplitude der überschiessenden Korrekturbewegungen bis zur definitiven Positionierung der Prüfmarke;
wobei vorzugsweise zusätzlich als Parameter ein Bild des Benutzers und/oder insbesondere einer vom Benutzer verwendeten Augenklappe registriert wird, und Informationen über das verwendete Auge oder den den Versuch durchführenden Benutzer zur Auswertung verwendet wird.

4. Verfahren nach einem der vorhergehenden Ansprüche, **dadurch gekennzeichnet, dass** entlang jeder Hauptachse zu Beginn des jeweiligen Testvorgangs ein Versatz (12) von 1/3, 1/2 und 2/3 der Distanz zwischen den beiden Aussenmarken (3, 4) vorgegeben wird, und wobei bevorzugtermassen die Anzahl der individuellen Testvorgänge und/oder der jeweilige Versatz (12) auf Basis von vorherigen Testserien und/oder vorangegangenen morphologischen Untersuchungen individuell angepasst sind.

5. Verfahren nach einem der vorhergehenden Ansprüche, **dadurch gekennzeichnet, dass** es sich bei den Aussenmarken (3, 4) und der Ausrichtungsmarke (5) um Striche oder Kreise mit einer Länge respektive Durchmesser im Bereich von 1-10 mm, vorzugsweise im Bereich von 2-4 mm handelt und im Falle von Strichen mit einer Strichdicke im Bereich von 0.5-5 mm, vorzugsweise im Bereich von 1-2.5 mm, und wobei alle Striche bevorzugtermassen während des ganzen Testvorgangs parallel zur auszumessenden Hauptachse ausgerichtet sind, wobei weiterhin vorzugsweise die Grösse der Aussenmarken und der Ausrichtungsmarke gleich sind, und weiterhin vorzugsweise in ihrer Grösse dem Sehvermögen der Testperson angepasst sind,
wobei vorzugsweise auf Basis von vorherigen Testserien oder vorherigen individuellen Testvorgängen oder vorangegangenen morphologischen Untersuchungen wenigstens einer der folgenden Einstellungsparameter individuell angepasst wird: die Grösse der Marken (3-5); der Kontrast auf dem Display (1); die Farbwahl auf dem Display (1); gegebenenfalls zusätzlich vorhandene Fixierhilfen, wobei diese Individualisierung, vorzugsweise für jedes spezifische Auge des Benutzers erfolgt.

6. Verfahren nach einem der vorhergehenden Ansprüche, **dadurch gekennzeichnet, dass** der Benutzer die Testserie mit nur einem Auge durchführt und/oder dass das elektronische Gerät (2) gewährleistet, dass die auf dem Display dargestellte Information nur von einem Auge wahrgenommen werden kann.

7. Verfahren nach einem der vorhergehenden Ansprüche, **dadurch gekennzeichnet, dass** der Benutzer das Verschieben der Ausrichtungsmarke durch eine direkte oder indirekte Interaktion mit dem elektronischen Gerät (2) kontrolliert, wobei diese Interaktion durch ein berührungsempfindliches Display, Körperbewegungen, insbesondere der Hand oder des Kopfes, oder durch Sprache, oder eine Kombination davon, erfolgen kann, wobei, wenn es sich beim Display (1) um ein berührungsempfindliches Display handelt, der Benutzer entweder die Ausrichtungsmarke (5) direkt durch berühren und verschieben der Ausrichtungsmarke (5) auf dem Display ausrichtet, oder unter Zuhilfenahme eines oder mehrerer Verschiebungsknöpfe, die auf dem Display (1) abgebildet sind.

8. Verfahren nach einem der vorhergehenden Ansprüche, **dadurch gekennzeichnet, dass** auf dem Display (1) in Abhängigkeit des ermittelten Sehvermögens eine qualitative Auswertung oder eine quantitative Auswertung ausgegeben wird, oder ein Hinweis, dass medizinische Hilfe aufgesucht werden soll,
oder dass die Anordnung der Aussenmarken und der Ausrichtungsmarke für die verschiedenen individuellen Testvorgänge individuell aufgrund der statistischen Auswertung der vorangegangenen Messungen angepasst ist.

9. Verfahren nach einem der vorhergehenden Ansprüche, **dadurch gekennzeichnet, dass** die in einer Testserie ermittelten Daten, gegebenenfalls in Kombination mit Informationen über den Benutzer, in personalisierter oder anonymisierter Form, an eine zentrale Stelle übermittelt werden, vorzugsweise über eine Internet- oder Mobiltelefon-Verbindung, und an dieser zentralen Stelle die Daten gespeichert, weiterverarbeitet, oder zur Information respektive Weiterbearbeitung an eine medizinische Betreuung weitergeleitet wird.

10. Verfahren nach einem der vorhergehenden Ansprüche, **dadurch gekennzeichnet, dass** das elektronische Gerät (2) zusätzlich über Sensoren, vorzugsweise in Form einer auf den Benutzer gerichteten Kamera, verfügt, und damit das Verhalten des Benutzers, insbesondere den Abstand des Benutzers zum Display (1), und/oder den Augenabstand, oder ob der Testvorgang ein- oder beidäugig durchgeführt wird, und in ersterem Fall, welches Auge dabei geschlossen ist, oder die Rotation des Displays um die Sehachse und/oder die Orthogonalität der Sehachse zur Fläche des Displays bestimmt und in die Datenanalyse einfliessen lässt oder über das Display (1) dem Benutzer einen Hinweis übermittelt, den die entsprechende Grösse auf den korrekten Wert einzustellen oder auf Basis dieser Informationen die auf dem Display erfolgte Darstellung von Aussenmarken und Ausrichtungsmarke anzupassen, vorzugsweise dynamisch anzupassen.

11. Verfahren nach einem der vorhergehenden Ansprüche, **dadurch gekennzeichnet, dass** es sich beim elektronischen Gerät (2) um ein tragbares Gerät, insbesondere ein PDA, Smartphone, Mobiltelefon, Tablet, Laptop, Smartwatch, Datenbrille oder Head-Mounted Display handelt.

12. Verfahren nach einem der vorhergehenden Ansprüche, **dadurch gekennzeichnet, dass** die Resultate von verschiedenen nacheinander durchgeführten Testserien, vorzugsweise von an unterschiedlichen Tagen durchgeführten Testserien, in ihrer Entwicklung vom elektronischen Gerät (2) und/oder einem zentralen Rechner relativ zueinander gewertet werden und die Entwicklung des Sehvermögens ermittelt wird, und vorzugsweise bei Erreichen eines Schwellenwerts einer aus den Testserien berechneten Grösse eine Warnung an den Benutzer ausgegeben wird und/oder über eine Schnittstelle an eine zentrale Stelle zur Kontaktaufnahme mit dem Benutzer übermittelt wird,
oder dass das elektronische Gerät den Benutzer im Rahmen eines vorgegebenen Zeitplans auffordert, jeweils zu einem bestimmten Zeitpunkt eine Testserie durchzuführen, wobei vorzugsweise in Abhängigkeit des Resultats von einer vorhergehenden Testserie und insbesondere je nach Änderungen der Resultate der unmittelbar vorhergehenden Testserie gegenüber den durch multiple vorhergehende Testserien ermittelten Basiswert, dem Benutzer eine erneute Testserie im Rahmen eines festen zeitlichen Schemas, vorzugsweise alle 3-14 Tage, insbesondere vorzugsweise alle 7 Tage, oder bei einer festgestellten Verschlechterung der Resultate bereits nach einem individuell verkürzten Zeitintervall empfohlen wird.

13. Verfahren nach einem der vorhergehenden Ansprüche, **dadurch gekennzeichnet, dass** bei der Ermittlung des Sehvermögens nicht allein durch das reine Sehvermögen verursachte Schwankungen, insbesondere tagesform-abhängige Schwankungen, weitgehend oder vollständig eliminiert werden, wobei dies vorzugsweise erfolgt durch entsprechende Berücksichtigung der Zeit, die der Benutzer für die Verschiebung der Ausrichtungsmarke (5) benötigt hat und/oder der Anzahl Richtungsänderungen bei der Verschiebung der Ausrichtungsmarke (5) durch den Benutzer;
oder dass insbesondere bei der Verwendung des ermittelten Sehvermögens zum Rückschluss auf den Zustand des Benutzers, vorzugsweise im Sinne eines Tests zur Reaktionsfähigkeit, einschliesslich Ermittlung des Einflusses von Drogen und/oder Medikamenten, nicht allein durch das reine Sehvermögen verursachte Schwankungen, insbesondere tagesform-abhängige Schwankungen, in der Analyse berücksichtigt werden, wobei dies vorzugsweise erfolgt durch entsprechende Berücksichtigung der Zeit, die der Benutzer für die Verschiebung der Ausrichtungsmarke (5) benötigt hat und/oder der Anzahl Richtungsänderungen bei der Verschiebung der Ausrichtungsmarke (5) durch den Benutzer, oder durch weitere durch Sensoren des Gerätes ermittelte Parameter.

14. Verfahren nach einem der vorgehenden Ansprüche, **dadurch gekennzeichnet, dass** die Messdaten des elektronischen Gerätes an einen zentralen Rechner gesendet werden und dort, unter Verwendung von Algorithmen, vorzugsweise:
i) der Varianzzunahme der Messpunktabstände zu einer Vergleichsmessung
ii) der Berücksichtigung von Patientencharakteristika, wie insbesondere Alter, Geschlecht, augenärztliche Befunde wie Sehstärke, Augen- und Systemerkrankungen;
iii) subjektiv erlebter Schwierigkeitsgrad der Testdurchführung, Testdauer, Anzahl und Amplitude der Steuerung und Korrektur der Testmarke und dem Verlauf der Testergebnisse, Mobilitätsverhalten des Benutzers, sowie allfälligen therapeutischen Massnahmen
analysiert werden, wobei die Resultate solcher Analysen als eine personalisierte Rückmeldung, vorzugsweise für jedes Auge individualisiert, aufbereitet und dem Benutzer zur Verfügung gestellt werden können und die Resultate dazu verwendet werden können, die Messanordnung zu individualisieren, wobei diese Individualisierung in einer Änderung der Messachsen, der Messpunkte, des Messintervalls und der Beurteilung der Messwerte bestehen kann.

15. Datenverarbeitungsprogramm zur Durchführung eines Verfahrens nach einem der Ansprüche 1-14 auf einem tragbaren elektronischen Gerät mit einem Display und einer Datenauswertungseinheit, wobei es sich vorzugsweise beim Display um ein berührungsempfindliches Display handelt, und wobei weiterhin vorzugsweise das elektronische Gerät zusätzlich über eine zum Benutzer gerichtete Kamera verfügt, wobei die Darstellung der Testaufgaben zwei- oder dreidimensional erfolgt, vorzugsweise in Form einer mobilen Applikation.

## Claims

1. A method of determining the eyesight of a user, in which the user, within the framework of a plurality of individual test procedures carried out one after the other in the sense of a series of tests in each case within the framework of an individual test procedure, two spaced-apart outer marks (3, 4) which are stationary within an individual test procedure and an alignment mark (5) which lies between the outer marks (3, 4) but not on a connecting straight line (6) of the outer marks (3, 4) are displayed on a display (1) of an electronic device (2),
and the user is requested by actuation of the electronic device (2) to displace the alignment mark (5) on the display (1) so far perpendicularly to the connecting straight line (6) of the outer marks (3, 4) until, as perceived by the user, the alignment mark (5) lies on the connecting straight line (6),
and subsequently, after displacement of the alignment mark (5) by the user, the electronic device (2) registers at least one of the following parameters: the effective distance (7) of the alignment mark (5) from the connecting straight line (6) after displacement of the alignment mark (5) by the user; the time required by the user for displacement of the alignment mark (5); the number of changes of direction during displacement of the alignment mark (5) by the user,
wherein at least two or at least three such individual test procedures are carried out, in which the external marks (3, 4) are arranged along the same main axis (8-11) or predetermined additional axes, and wherein at least two different main axes are measured in the course of the test series,
and wherein the at least one registered parameter or, in the case of measurement of several parameters, a combination of the registered parameters, is used as a measure for determining the visual acuity from the individual test procedures of the test series,
**characterised in that**
in the context of the test series, at least two of the test operations are carried out along the vertical main axis (8) of the display (1) and two test operations are carried out along the horizontal main axis (9) of the display (1), and in each case at least two of the test operations are carried out along at least one of the two diagonal main axes (10, 11)
and **in that** the test operations along the same main axis are each started with a different offset (12) of the alignment mark (5) along the connecting straight line (6).

2. Method according to claim 1, **characterised in that**, within the framework of a test series, at least three test procedures are carried out along the vertical main axis (8) and at least three test procedures are carried out along the horizontal main axis (9), and in each case at least two or at least three test procedures are carried out along at least one of the two diagonal main axes (10, 11).
wherein preferably in the case of weaknesses of the user registered along certain main axes (8-11), these main axes are measured particularly or subsequently only substantially exclusively, and/or wherein conclusions are drawn from the visual ability determined along certain main axes (8-11) as to the abilities of certain areas in the retinal region, wherein these areas are defined in the polar grid of a polar coordinate system as circular sectors or circular ring sectors.

3. Method according to one of the preceding claims, **characterised in that** when registering the parameter of the effective distance (7) of the alignment mark (5) from the connecting line (6) after displacement of the alignment mark (5) by the user, the mean value and/or the standard deviation of the magnitude of the effective distance (7) of the test procedures along the same principal axis is used as a measure for determining the visual acuity;
or when registering the parameter of the time taken by the user to move the alignment mark (5), the mean and/or the standard deviation of the total time between the appearance of the marks (3-5) and the acknowledgement by the user of the end of the individual test procedure is used as the measure for determining the visual acuity;
or when registering the parameter of the number of directional changes in the displacement of the alignment marker (5) by the user, the mean value and/or the standard deviation of the number of directional changes in the individual test procedures is used, or the sum of the directional changes along a certain main axis, or the magnitude and/or amplitude of the overshooting correction movements until the definitive positioning of the test marker;
wherein preferably additionally as a parameter an image of the user and/or in particular of an eye patch used by the user is registered, and information about the eye used or the user performing the test is used for evaluation.

4. Method according to one of the preceding claims, **characterised in that** an offset (12) of 1/3, 1/2 and 2/3 of the distance between the two outer marks (3, 4) is predetermined along each principal axis at the beginning of the respective test procedure, and wherein preferably the number of individual test procedures and/or the respective offset (12) are individually adapted on the basis of previous test series and/or previous morphological examinations.

5. Method according to one of the preceding claims, **characterized in that** the outer marks (3, 4) and the alignment mark (5) are strokes or circles with a length or diameter, respectively, in the range of 1-10 mm, preferably in the range of 2-4 mm, and in the case of strokes with a stroke thickness in the range of 0. 5-5 mm, preferably in the range of 1-2.5 mm, and wherein all strokes are preferably aligned parallel to the main axis to be measured during the entire test procedure, wherein furthermore preferably the size of the outer marks and the alignment mark are the same, and furthermore preferably are adapted in their size to the vision of the test person,
wherein preferably on the basis of previous test series or previous individual test procedures or previous morphological examinations at least one of the following adjustment parameters is individually adapted: the size of the marks (3-5); the contrast on the display (1); the choice of colour on the display (1); if necessary additionally present fixation aids, wherein this individualisation is preferably effected for each specific eye of the user.

6. Method according to one of the preceding claims, **characterised in that** the user performs the test series with only one eye and/or that the electronic device (2) ensures that the information shown on the display can only be perceived by one eye.

7. Method according to any one of the preceding claims, **characterized in that** the user controls the displacement of the alignment mark by a direct or indirect interaction with the electronic device (2), which interaction may be performed by a touch-sensitive display, body movements, in particular of the hand or head, or by speech, or a combination thereof, wherein, if the display (1) is a touch-sensitive display, the user either aligns the alignment mark (5) directly by touching and sliding the alignment mark (5) on the display, or with the aid of one or more sliding buttons depicted on the display (1).

8. Method according to one of the preceding claims, **characterised in that** a qualitative evaluation or a quantitative evaluation is output on the display (1) as a function of the determined visual acuity, or an indication that medical help should be sought,
or **in that** the arrangement of the outer marks and the alignment mark for the various individual test procedures is individually adapted on the basis of the statistical evaluation of the preceding measurements.

9. Method according to one of the preceding claims, **characterised in that** the data determined in a test series, if necessary in combination with information about the user, is transmitted in personalised or anonymised form to a central point, preferably via an internet or mobile telephone connection, and at this central point the data is stored, further processed, or forwarded to medical care for information or further processing.

10. Method according to one of the preceding claims, **characterised in that** the electronic device (2) additionally has sensors, preferably in the form of a camera directed at the user, and thus the behaviour of the user, in particular the distance of the user from the display (1), and/or the interpupillary distance, or whether the test procedure is carried out monocularly or binocularly, and in the former case, which eye is closed in the process, or the rotation of the display about the visual axis and/or the orthogonality of the visual axis to the surface of the display is determined and included in the data analysis, or an indication is transmitted to the user via the display (1) to adjust the corresponding size to the correct value or to adjust, preferably dynamically adjust, the representation of outer marks and alignment marks on the display on the basis of this information.

11. Method according to one of the preceding claims, **characterised in that** the electronic device (2) is a portable device, in particular a PDA, smartphone, mobile phone, tablet, laptop, smartwatch, data glasses or head-mounted display.

12. Method according to one of the preceding claims, **characterised in that** the results of different test series carried out one after the other, preferably of test series carried out on different days, are evaluated in their development relative to one another by the electronic device (2) and/or a central computer and the development of the vision is determined, and preferably when a threshold value of a quantity calculated from the test series is reached, a warning is issued to the user and/or is transmitted via an interface to a central point for contacting the user,
or **in that** the electronic device prompts the user within the framework of a predetermined time schedule to carry out a test series in each case at a specific time, in which case, preferably as a function of the result of a preceding test series and in particular depending on changes in the results of the immediately preceding test series compared with the base value determined by multiple preceding test series, a renewed test series is recommended to the user within the framework of a fixed time schedule, preferably every 3-14 days, in particular preferably every 7 days, or in the case of a detected deterioration in the results already after an individually shortened time interval.

13. Method according to one of the preceding claims, **characterised in that**, when determining the visual acuity, fluctuations which are not caused solely by the pure visual acuity, in particular fluctuations which are dependent on the form of day, are largely or completely eliminated, this preferably taking place by correspondingly taking into account the time which the user has required for the displacement of the alignment mark (5) and/or the number of changes in direction during the displacement of the alignment mark (5) by the user;
or **in that**, in particular when the determined vision is used to draw conclusions about the state of the user, preferably in the sense of a test for responsiveness, including determination of the influence of drugs and/or medications, fluctuations which are not caused solely by the pure vision, in particular fluctuations which are dependent on the form of the day, are taken into account in the analysis, this preferably taking place by correspondingly taking into account the time which the user has required for the displacement of the alignment mark (5) and/or the number of changes in direction when the alignment mark (5) is displaced by the user, or by further parameters determined by sensors of the device.

14. Method according to one of the preceding claims, **characterized in that** the measurement data of the electronic device are sent to a central computer and there, using algorithms, preferably:
i) the variance increase of the measuring point distances to a comparison measurement
ii) the consideration of patient characteristics, such as in particular age, gender, ophthalmological findings such as visual acuity, ocular and systemic diseases
iii) subjectively experienced difficulty of test performance, test duration, number and amplitude of control and correction of the test mark and the course of the test results, mobility behaviour of the user, as well as possible therapeutic measures the results of such analyses can be processed as a personalised feedback, preferably individualised for each eye, and made available to the user and the results can be used to individualise the measurement set-up, whereby this individualisation can consist in a change of the measurement axes, the measurement points, the measurement interval and the assessment of the measurement values.

15. Data processing program for carrying out a method according to any one of claims 1-14 on a portable electronic device for having a display and a data evaluation unit, wherein the display is preferably a touch-sensitive display, and wherein furthermore preferably the electronic device additionally has a camera directed towards the user, wherein the display of the test tasks is two- or three-dimensional, preferably in the form of a mobile application.

## Revendications

1. Procédé pour déterminer la vue d'un utilisateur, dans lequel l'utilisateur, dans le cadre de plusieurs procédures de test individuelles exécutées les unes après les autres au sens d'une série de tests, à chaque fois dans le cadre d'une procédure de test individuelle, deux marques extérieures (3, 4) espacées l'une de l'autre et stationnaires dans une procédure de test individuelle et une marque d'alignement (5) située entre les marques extérieures (3, 4) mais pas sur une droite de liaison (6) des marques extérieures (3, 4) sont affichées sur un écran (1) d'un dispositif électronique (2),
et il est demandé à l'utilisateur, par actionnement du dispositif électronique (2), de déplacer la marque d'alignement (5) sur l'affichage (1) jusqu'à ce qu'elle soit perpendiculaire à la ligne droite de connexion (6) des marques extérieures (3, 4), jusqu'à ce que, selon la perception de l'utilisateur, la marque d'alignement (5) se trouve sur la ligne droite de connexion (6),
et ensuite, après le déplacement de la marque d'alignement (5) par l'utilisateur, le dispositif électronique (2) enregistre au moins un des paramètres suivants : la distance effective (7) de la marque d'alignement (5) par rapport à la ligne droite de connexion (6) après le déplacement de la marque d'alignement (5) par l'utilisateur ; le temps requis par l'utilisateur pour le déplacement de la marque d'alignement (5) ; le nombre de changements de direction pendant le déplacement de la marque d'alignement (5) par l'utilisateur,
dans lequel au moins deux ou au moins trois de ces procédures de test individuelles sont effectuées, dans lesquelles les marques extérieures (3, 4) sont disposées le long du même axe principal (8-11) ou d'axes supplémentaires prédéterminés, et dans lequel au moins deux axes principaux différents sont mesurés au cours de la série de tests,
et dans lequel le au moins un paramètre enregistré ou, dans le cas de la mesure de plusieurs paramètres, une combinaison des paramètres enregistrés, est utilisé comme mesure pour déterminer l'acuité visuelle à partir des procédures de test individuelles de la série de tests,
**caractérisé en ce que**
dans le cadre de la série de tests, au moins deux des opérations de test sont effectuées le long de l'axe principal vertical (8) de l'écran (1) et deux opérations de test sont effectuées le long de l'axe principal horizontal (9) de l'écran (1), et dans chaque cas, au moins deux des opérations de test sont effectuées le long d'au moins un des deux axes principaux diagonaux (10, 11)
et **en ce que** les opérations de test le long du même axe principal sont chacune démarrées avec un décalage différent (12) de la marque d'alignement (5) le long de la ligne droite de connexion (6).

2. Procédé selon la revendication 1, **caractérisé en ce que**, dans le cadre d'une série de tests, au moins trois procédures de test sont effectuées le long de l'axe principal vertical (8) et au moins trois procédures de test sont effectuées le long de l'axe principal horizontal (9), et dans chaque cas, au moins deux ou au moins trois procédures de test sont effectuées le long d'au moins un des deux axes principaux diagonaux (10, 11),
dans lequel, de préférence, en cas de faiblesses de l'utilisateur enregistrées le long de certains axes principaux (8-11), ces axes principaux sont mesurés particulièrement ou ensuite seulement sensiblement exclusivement, et/ou dans lequel des conclusions sont tirées de la capacité visuelle déterminée le long de certains axes principaux (8-11) quant aux capacités de certaines zones dans la région de la rétine, dans lequel ces zones sont définies dans la grille polaire d'un système de coordonnées polaires comme des secteurs circulaires ou des secteurs d'anneau circulaire.

3. Procédé selon l'une des revendications précédentes, **caractérisé en ce que** lors de l'enregistrement du paramètre de la distance effective (7) de la marque d'alignement (5) par rapport à la ligne de liaison (6) après déplacement de la marque d'alignement (5) par l'utilisateur, la valeur moyenne et/ou l'écart-type de la grandeur de la distance effective (7) des procédures de test le long du même axe principal est utilisée comme mesure pour déterminer l'acuité visuelle ;
ou lors de l'enregistrement du paramètre du temps pris par l'utilisateur pour déplacer la marque d'alignement (5), la moyenne et/ou l'écart type du temps total entre l'apparition des marques (3-5) et la confirmation par l'utilisateur de la fin de la procédure de test individuelle est utilisée comme mesure pour déterminer l'acuité visuelle ;
ou lors de l'enregistrement du paramètre du nombre de changements de direction dans le déplacement du marqueur d'alignement (5) par l'utilisateur, la valeur moyenne et/ou l'écart type du nombre de changements de direction dans les procédures de test individuelles est utilisé, ou la somme des changements de direction le long d'un certain axe principal, ou la magnitude et/ou l'amplitude des mouvements de correction de dépassement jusqu'au positionnement définitif du marqueur de test ;
dans lequel, de préférence, une image de l'utilisateur et/ou en particulier d'un cache-œil utilisé par l'utilisateur est enregistrée en tant que paramètre supplémentaire et les informations sur l'œil utilisé ou l'utilisateur effectuant le test sont utilisées pour l'évaluation.

4. Procédé selon l'une des revendications précédentes, **caractérisé en ce qu'**un décalage (12) de 1/3, 1/2 et 2/3 de la distance entre les deux marques extérieures (3, 4) est prédéterminé le long de chaque axe principal au début de la procédure de test respective, et dans lequel de préférence le nombre de procédures de test individuelles et/ou le décalage respectif (12) sont adaptés individuellement sur la base de séries de test précédentes et/ou d'examens morphologiques précédents.

5. Procédé selon l'une des revendications précédentes, **caractérisé en ce que** les marques extérieures (3, 4) et la marque d'alignement (5) sont des traits ou des cercles dont la longueur ou le diamètre est respectivement compris entre 1 et 10 mm, de préférence entre 2 et 4 mm, et dans le cas de traits dont l'épaisseur est comprise entre 0,5 et 5 mm, de préférence entre 0,5 et 0,5 mm, 5-5 mm, de préférence dans la plage de 1-2,5 mm, et dans lequel tous les traits sont de préférence alignés parallèlement à l'axe principal à mesurer pendant toute la procédure de test, dans lequel en outre, de préférence, la taille des marques extérieures et de la marque d'alignement est la même, et en outre, de préférence, leur taille est adaptée à la vision de la personne testée,
dans lequel, de préférence, sur la base de séries de tests antérieures ou de procédures de tests individuels antérieures ou d'examens morphologiques antérieurs, au moins l'un des paramètres de réglage suivants est adapté individuellement : la taille des marques (3-5) ; le contraste sur l'écran (1) ; le choix de la couleur sur l'écran (1) ; si nécessaire, des aides de fixation supplémentaires présentes, dans lequel cette individualisation est de préférence effectuée pour chaque œil spécifique de l'utilisateur.

6. Procédé selon l'une des revendications précédentes, **caractérisé en ce que** l'utilisateur effectue la série de tests avec un seul œil et/ou que le dispositif électronique (2) fait en sorte que les informations affichées sur l'écran ne puissent être perçues que par un seul œil.

7. Procédé selon l'une quelconque des revendications précédentes, **caractérisé en ce que** l'utilisateur commande le déplacement de la marque d'alignement par une interaction directe ou indirecte avec le dispositif électronique (2), laquelle interaction peut être réalisée par un écran tactile, des mouvements corporels, en particulier de la main ou de la tête, ou par la parole, ou une combinaison de ceux-ci, dans lequel, si l'écran (1) est un écran tactile, l'utilisateur aligne la marque d'alignement (5) soit directement en touchant et en faisant glisser la marque d'alignement (5) sur l'écran, soit à l'aide d'un ou plusieurs boutons coulissants représentés sur l'écran (1).

8. Procédé selon l'une des revendications précédentes, **caractérisé en ce qu'**une évaluation qualitative ou une évaluation quantitative est émise sur l'écran (1) en fonction de l'acuité visuelle déterminée, ou une indication qu'une aide médicale doit être recherchée,
ou **en ce que** la disposition des marques extérieures et de la marque d'alignement pour les différentes procédures de test individuelles est adaptée individuellement sur la base de l'évaluation statistique des mesures précédentes.

9. Procédé selon l'une des revendications précédentes, **caractérisé en ce que** les données déterminées dans une série de tests, le cas échéant en combinaison avec des informations sur l'utilisateur, sont transmises sous une forme personnalisée ou anonymisée à un point central, de préférence par une connexion Internet ou de téléphonie mobile, et à ce point central, les données sont stockées, traitées ultérieurement ou transmises aux soins médicaux pour information ou traitement ultérieur.

10. Procédé selon l'une des revendications précédentes, **caractérisé en ce que** le dispositif électronique (2) comporte en outre des capteurs, de préférence sous la forme d'une caméra dirigée vers l'utilisateur, et donc le comportement de l'utilisateur, en particulier la distance de l'utilisateur par rapport à l'écran (1), et/ou la distance inter pupillaire, ou si la procédure de test est effectuée de manière monoculaire ou binoculaire, et dans le premier cas, quel œil est fermé dans le processus, ou la rotation de l'écran autour de l'axe visuel et/ou l'orthogonalité de l'axe visuel par rapport à la surface de l'écran est déterminée et incluse dans l'analyse des données, ou une indication est transmise à l'utilisateur par l'intermédiaire de l'écran (1) pour ajuster la taille correspondante à la valeur correcte ou pour ajuster, de préférence dynamiquement, la représentation des marques extérieures et des marques d'alignement sur l'écran sur la base de ces informations.

11. Procédé selon l'une des revendications précédentes, **caractérisé en ce que** le dispositif électronique (2) est un dispositif portable, notamment un PDA, un smartphone, un téléphone mobile, une tablette, un ordinateur portable, une smart Watch, des lunettes de données ou un affichage monté sur la tête.

12. Procédé selon l'une des revendications précédentes, **caractérisé en ce que** les résultats de différentes séries de tests effectuées l'une après l'autre, de préférence de séries de tests effectuées à des jours différents, sont évalués dans leur évolution les uns par rapport aux autres par le dispositif électronique (2) et/ou un ordinateur central et l'évolution de la vision est déterminée, et de préférence lorsqu'une valeur seuil d'une grandeur calculée à partir des séries de tests est atteinte, un avertissement est émis à l'intention de l'utilisateur et/ou est transmis via une interface à un point central pour contacter l'utilisateur,
ou **en ce que** le dispositif électronique invite l'utilisateur, dans le cadre d'un calendrier prédéterminé, à effectuer une série de tests à un moment précis, auquel cas, de préférence en fonction du résultat d'une série de tests précédente, et en particulier en fonction des modifications des résultats de la série de tests immédiatement précédente par rapport à la valeur de base déterminée par plusieurs séries de tests précédentes, une nouvelle série de tests est recommandée à l'utilisateur dans le cadre d'un calendrier fixe, de préférence tous les 3 à 14 jours, en particulier de préférence tous les 7 jours, ou en cas de détérioration détectée des résultats déjà après un intervalle de temps raccourci individuellement.

13. Procédé selon l'une des revendications précédentes, **caractérisé en ce que**, lors de la détermination de l'acuité visuelle, les fluctuations qui ne sont pas causées uniquement par l'acuité visuelle pure, en particulier les fluctuations qui dépendent de la forme du jour, sont largement ou complètement éliminées, ceci ayant lieu de préférence en prenant en compte de manière correspondante le temps que l'utilisateur a exigé pour le déplacement de la marque d'alignement (5) et/ou le nombre de changements de direction pendant le déplacement de la marque d'alignement (5) par l'utilisateur ;
ou **en ce que**, en particulier lorsque la vision déterminée est utilisée pour tirer des conclusions sur l'état de l'utilisateur, de préférence dans le sens d'un test de réactivité, y compris la détermination de l'influence de drogues et/ou de médicaments, les fluctuations qui ne sont pas causées uniquement par la vision pure, en particulier les fluctuations qui dépendent de la forme du jour, sont prises en compte dans l'analyse, ce qui se fait de préférence en prenant en compte de manière correspondante le temps nécessaire à l'utilisateur pour déplacer la marque d'alignement (5) et/ou le nombre de changements de direction lors du déplacement de la marque d'alignement (5) par l'utilisateur, ou d'autres paramètres déterminés par des capteurs du dispositif.

14. Procédé selon l'une des revendications précédentes, **caractérisé en ce que** les données de mesure du dispositif électronique sont envoyées à un ordinateur central et là, à l'aide d'algorithmes, de préférence :
i) l'augmentation de la variance des distances des points de mesure par rapport à une mesure de comparaison
ii) la prise en compte des caractéristiques du patient, comme en particulier l'âge, le sexe, les résultats ophtalmologiques tels que l'acuité visuelle, les maladies oculaires et systémiques
iii) la difficulté subjectivement ressentie de la réalisation du test, la durée du test, le nombre et l'amplitude du contrôle et de la correction de la marque du test et le déroulement des résultats du test, le comportement de mobilité de l'utilisateur, ainsi que les mesures thérapeutiques éventuelles.
les résultats de ces analyses peuvent être traités sous la forme d'un retour d'information personnalisé, de préférence individualisé pour chaque œil, et mis à la disposition de l'utilisateur, et les résultats peuvent être utilisés pour individualiser le dispositif de mesure, cette individualisation pouvant consister en une modification des axes de mesure, des points de mesure, de l'intervalle de mesure et de l'évaluation des valeurs de mesure.

15. Programme de traitement de données pour la mise en œuvre d'un procédé selon l'une quelconque des revendications 1 à 14 sur un dispositif électronique portable pour avoir un écran et une unité d'évaluation de données, dans lequel l'écran est de préférence un écran tactile, et dans lequel en outre de préférence le dispositif électronique a en plus une caméra dirigée vers l'utilisateur, dans lequel l'affichage des tâches de test est en deux ou trois dimensions, de préférence sous la forme d'une application mobile.
